# EUROPEAN PATENT SPECIFICATION

(11) **EP 4 751 776 B1**
(45) Date of publication and mention of the grant of the patent: **11.11.2026**
(21) Application number: 26164598.0
(22) Date of filing: 29.06.2016
(51) Int. Cl.: A61K 9/20, A61K 9/00, A61K 9/24, A61K 9/28, A61K 31/513, A61K 31/675

(54) **TABLETS COMPRISING EMTRICITABINE AND TENOFOVIR ALAFENAMIDE**
TABLETTEN ENTHALTEND EMTRICITABIN UND TENOFOVIR ALAFENAMID
COMPRIMÉS CONTENANT DE L'EMTRICITABINE ET DU TÉNOFOVIR ALAFÉNAMIDE

(30) Priority: 30.06.2015 US 201562187113 P; 22.02.2016 US 201662298373 P; 29.02.2016 US 201662301429 P; 01.04.2016 US 201662317286 P
(43) Date of publication of application: 03.06.2026
(60) Divisional application: 26173766.2 / 4 755 454; 26175955.9 / 4 759 366
(62) Document number(s) of the earlier application(s) in accordance with Art. 76 EPC: 24216441.6 / 4 527 467; 23162978.3 / 4 233 846; 22176537.3 / 4 070 788; 19199257.7 / 3 607 939; 16739625.8 / 3 316 868
(73) Proprietor: Gilead Sciences, Inc., Foster City, CA 94404 (US)
(72) Inventor: KOZIARA, Joanna M., Foster City, CA 94404 (US); MCCALLISTER, Scott, Foster City, CA 94404 (US)
(74) Representative: Carpmaels & Ransford LLP

(56) References cited:
- WO-A1-2013/116720
- WO-A1-2015/022351
- WO-A1-2015/196116
- GILEAD: "Gilead Submits New Drug Application to U.S. Food and Drug Administration for Fixed-Dose Combination of Emtricitabine/Tenofovir Alafenamide for HIV Treatment", PRESS RELEASE, 7 April 2015 (2015-04-07), pages 1 - 2, XP055757389, Retrieved from the Internet <URL:https://www.gilead.com/news/news-details/2015/gilead-submits-new-drug-application-to-us-food-and-drug-administration-for-fixed-dose-combination-of-emtricitabinetenofovir-alafenamide-for-hiv-treatment> [retrieved on 20201207]

## Description

### TECHNICAL FIELD

Pharmaceutical formulations suitable for treating viral infections such as HIV are disclosed, in particular tablets including emtricitabine and tenofovir alafenamide.

### BACKGROUND

Human immunodeficiency virus, type 1 (HIV-1) infection is a life-threatening and serious disease of major public health significance, with approximately 35 million people infected worldwide (Joint United Nations Programme on HIV/AIDS (UNAIDS). Global report: UNAIDS report on the global AIDS epidemic, 2013). Standard of care for the treatment of HIV-1 infection uses combination antiretroviral therapy (ART) to suppress viral replication to below detectable limits, increase CD4 cell counts, and halt disease progression.

The success of potent and well-tolerated ART means that morbidity and mortality in the HIV-infected population is increasingly driven by non-AIDS associated comorbidities. Clinical attention has become more focused on optimizing tolerability, long-term safety, and adherence (Costagliola D. Demographics of HIV and aging. Curr. Opin. HIV AIDS, 2014, 9(4), 294). There remains a significant medical need for safe and effective new therapies that take into consideration the aging patient population, non-HIV-related comorbidities, virologic resistance, and regimen simplification. WO2013/116720 discloses combination therapy comprising tenofovir alafenamide hemifumarate and cobicistat for use in the treatment of viral infections. A Gilead Press Release of 2015 titled 'Gilead Submits New Drug Application to U.S. Food and Drug Administration for Fixed-Dose Combination of Emtricitabine/Tenofovir Alafenamide for HIV Treatment' (XP055757389), discloses a fixed dose combination of 200mg emtricitabine and 25mg tenofovir alafenamide.

### SUMMARY

The inventors have successfully formulated a tablet according to claim 1 containing tenofovir alafenamide and emtricitabine. This tablet may be suitable for use in medicine, and in particular in treating viral infections such as HIV.

The inventors have found that it is possible to formulate tablets that are intended to be pharmaceutically acceptable (i.e. pharmacologically efficacious and physically acceptable) while reducing the total amount of excipients necessary to achieve stability. The tablets of the invention contain two (and only two) active pharmaceutical ingredients: tenofovir alafenamide or a pharmaceutically acceptable salt thereof and emtricitabine or a pharmaceutically acceptable salt thereof.

The inventors have demonstrated that it is possible to formulate stable compositions containing tenofovir alafenamide and emtricitabine that exhibit acceptable stability. Accordingly, in another aspect the invention provides a tablet comprising (a) tenofovir alafenamide or a pharmaceutically acceptable salt thereof in the amount specified in claim 1, and (b) emtricitabine or a pharmaceutically acceptable salt thereof in the amount specified in claim 1, where the total quantity of degradation products derived from the tenofovir alafenamide or the pharmaceutically acceptable salt thereof is less than 3% after storage for one month at 40°C/75% RH in open conditions.

The inventors have also found that there is a relationship between the stability of tenofovir alafenamide and the concentration of tenofovir alafenamide within a given composition. The invention provides a tablet comprising tenofovir alafenamide or a pharmaceutically acceptable salt thereof wherein the proportion of tenofovir alafenamide in the tablet is from 4% to 9% by weight.

In another aspect, a kit comprising (a) a tablet according to the claims comprising tenofovir alafenamide or a pharmaceutically acceptable salt thereof, and emtricitabine or a pharmaceutically acceptable salt thereof, and (b) a desiccant (e.g. silica gel) is provided. In another aspect, a kit comprising (a) a tablet according to the claims comprising tenofovir alafenamide or a pharmaceutically acceptable salt thereof, and emtricitabine or a pharmaceutically acceptable salt thereof, and (b) a desiccant (e.g. silica gel) is provided, wherein the total quantity of degradation products derived from the tenofovir alafenamide or the pharmaceutically acceptable salt thereof is less than 2% after storage for twelve months at 30°C/75% RH. In another aspect, a kit comprising (a) a tablet according to the claims comprising tenofovir alafenamide or a pharmaceutically acceptable salt thereof, and emtricitabine or a pharmaceutically acceptable salt thereof, and (b) a desiccant (e.g. silica gel) is provided, wherein the total quantity of degradation products derived from the tenofovir alafenamide or the pharmaceutically acceptable salt thereof is less than 2.5% after storage for three months at 40°C/75% RH.

Methods of producing tablets are also disclosed (but not claimed), as discussed in more detail below.

In addition, tablets for use in methods for treating patients are provided, which are also discussed in more detail below.

### BRIEF DESCRIPTION OF DRAWINGS

Figure 1 shows the percent of degradation of tenofovir alafenamide hemifumarate as a function of drug load.
Figures 2A and B illustrate the impact on the stability of tenofovir alafenamide hemifumarate of the presence of (i) emtricitabine, and (ii) emtricitabine and rilpivirine HCl. Figure 2A shows the total degradation of tenofovir alafenamide hemifumarate at 40°C/75% RH under open conditions, and Figure 2B shows the total degradation of tenofovir alafenamide hemifumarate at 60°C under closed conditions.
Figure 3 is a flow diagram illustrating the preparation of a tablet formulation of emtricitabine and tenofovir alafenamide hemifumarate.

### DETAILED DESCRIPTION

The tablets consist of two (i.e. only two) active pharmaceutical ingredients: tenofovir alafenamide (or a pharmaceutically acceptable salt thereof), and emtricitabine (or a pharmaceutically acceptable salt thereof).

### Tenofovir alafenamide

Tenofovir alafenamide (TAF) is a nucleotide reverse transcriptase inhibitor having the formula below (see WO02/08241 A2):

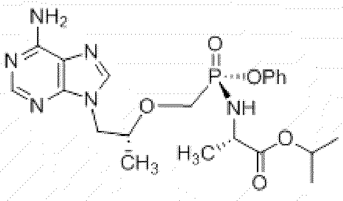

Its IUPAC name is (*S*)-isopropyl-2-(((*S*)-((((*R*)-1-(6-amino-9*H*-purin-9-yl)propan-2-yl)oxy)methyl)(phenoxy)phosphoryl)amino)propanoate. It is also referred to as {9-[(R)-2-[[(S)-[[(S)-1-(isopropoxycarbonyl)ethyl]amino]phenoxyphosphinyl]-methoxy]propyl]adenine}.

Tablets of the invention include tenofovir alafenamide, usually in the form of a pharmaceutically acceptable salt. Tenofovir alafenamide can be present within a tablet in solvated or unsolvated form, and references to "tenofovir alafenamide" include both of these forms. In particular, tenofovir alafenamide may be associated with fumarate, such as monofumarate or hemifumarate. Typically, tenofovir alafenamide is in the form of tenofovir alafenamide hemifumarate having the formula below (see WO 2013/025788 A1):

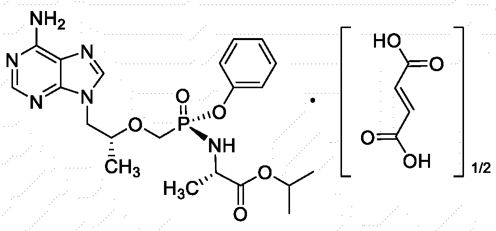

As used herein, and in the absence of a specific reference to a particular pharmaceutically acceptable salt and/or solvate of tenofovir alafenamide, any dosages, whether expressed in e.g. milligrams or as a % by weight, should be taken as referring to the amount of tenofovir alafenamide, *i.e.* the amount of:

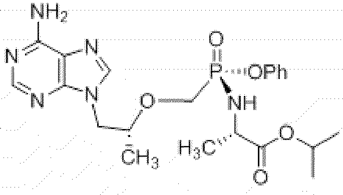

For example, therefore, a reference to "25 mg tenofovir alafenamide or a pharmaceutically acceptable salt and/or solvate thereof" means an amount of tenofovir alafenamide or a pharmaceutically acceptable salt and/or solvate thereof which provides the same amount of tenofovir alafenamide as 25 mg of tenofovir alafenamide *free base.*

The amount of tenofovir alafenamide in a tablet provided herein is an amount of tenofovir alafenamide or a pharmaceutically acceptable salt thereof which provides the same amount of tenofovir alafenamide as 25 mg of tenofovir alafenamide free base e.g. as about 28 mg of tenofovir alafenamide hemifumarate.

### Emtricitabine

Emtricitabine (FTC) is a nucleoside reverse transcriptase inhibitor having the formula below:

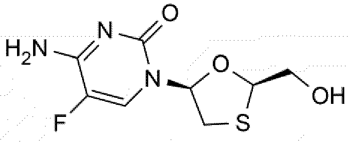

Its IUPAC name is 4-amino-5-fluoro-1-[(2*R*,5*S*)-2-(hydroxymethyl)-1,3-oxathiolan-5-yl]-1,2-dihydropyrimidin-2-one. It is also referred to as 5-fluoro-1-[(2R,5S)-2-(hydroxymethyl)-1,3-oxathiolan-5-yl]cytosine. It is currently authorised as part of EMTRIVA^{®} (emtricitabine 200 mg), TRUVADA^{®} (emtricitabine 200 mg, tenofovir disoproxil fumarate 300 mg), ATRIPLA^{®} (emtricitabine 200 mg, efavirenz 600 mg, tenofovir disoproxil fumarate 300 mg) and STRIBILD^{®} (emtricitabine 200 mg, cobicistat 150 mg, tenofovir disoproxil fumarate 300 mg, elvitegravir 150 mg) and COMPLERA^{®}/EVIPLERA^{®}.

Tablets disclosed herein include emtricitabine, optionally as a pharmaceutically acceptable salt. Emtricitabine can be present within a tablet in solvated or unsolvated form, and references to "emtricitabine" include both of these forms. Typically, emtricitabine is present as a free base.

As used herein, and in the absence of a specific reference to a particular pharmaceutically acceptable salt and/or solvate of emtricitabine, any dosages, whether expressed in *e.g*. milligrams or as a % by weight, should be taken as referring to the amount of emtricitabine, *i.e.* the amount of:

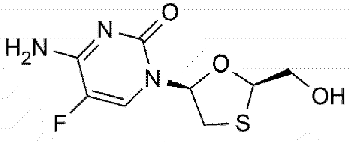

For example, therefore, a reference to "200 mg emtricitabine or a pharmaceutically acceptable salt and/or solvate thereof" means an amount of emtricitabine or a pharmaceutically acceptable salt and/or solvate thereof which provides the same amount of emtricitabine as 200 mg of emtricitabine *free base.*

The amount of emtricitabine in a tablet provided herein is an amount of emtricitabine or a pharmaceutically acceptable salt thereof which provides the same amount of emtricitabine as 200 mg of emtricitabine free base.

### Solid oral dosage forms

The inventors have successfully formulated emtricitabine and tenofovir alafenamide in a single, stable tablet that is intended to be pharmacologically efficacious and physically acceptable. The tablets disclosed herein are intended for pharmaceutical use in human subjects. Accordingly, they must be of an appropriate size and weight for oral human administration, in addition to being therapeutically efficacious.

In addition to the clinical benefits described above may result from the use of tenofovir alafenamide, the dosage forms of the present invention may afford further advantages. The inventors have determined that it is possible to formulate emtricitabine and tenofovir alafenamide into a tablet which has a total weight of less than 500 mg, or less than 400 mg. TRUVADA^{®} has a total weight of about 1000 mg. The provision of a relatively small tablet represents a clinical advantage because it may be expected to increase patient convenience and thus compliance as compared to larger dosage forms which are more burdensome for patients to swallow. By way of comparison, TRUVADA^{®} contains about 500 mg of excipients, whereas the presently disclosed tablets comprise less than 300 mg of excipients, such as less than 250 mg of excipients, or 200 mg of excipients or less. In certain embodiments, the solid oral dosage forms disclosed herein include between 100 mg and 200 mg of excipients, or between 100 mg and 175 mg of excipients, or between 100 mg and 150 mg of excipients. In certain embodiments, solid oral dosage forms disclosed herein comprise between 120 mg and 150 mg of excipients. The tablets comprise as active ingredients (a) an amount of tenofovir alafenamide or a pharmaceutically acceptable salt thereof which provides the same amount of tenofovir alafenamide as 25 mg tenofovir alafenamide free base, and (b) an amount of emtricitabine or a pharmaceutically acceptable salt thereof which provides the same amount of emtricitabine as 200 mg emtricitabine free base. The tablets may comprise as active ingredients (a) 28 mg tenofovir alafenamide hemifumarate, and (b) 200 mg emtricitabine.

The solid oral dosage forms described herein are in the form of a tablet. In particular embodiments, they are in the form of a monolayer tablet. In particular embodiments, they may be in the form of a multilayer tablet. This is because the inventors have found that the use of multilayer tablets may assist in optimizing the properties of the dosage form, particularly the stability (*e.g.* of tenofovir alafenamide). They have also discovered that the use of multilayer tablets may affect the dissolution profile of one or more of the active ingredients within the dosage form, and is therefore likely to have an impact on the *in vivo* pharmacokinetics of the dosage form. The provision of a tablet with particular pharmacokinetic parameters, e.g. pharmacokinetic parameters that are bioequivalent with existing medicines (or medicines at an advanced stage of the regulatory procedure) is a particular advantage that may be afforded by the present invention. Achieving bioequivalence may require the use of a multilayer tablet.

In one embodiment, a tablet comprising (a) tenofovir alafenamide or a pharmaceutically acceptable salt thereof, and (b) emtricitabine or a pharmaceutically acceptable salt thereof according to the appended claims is provided. In one embodiment, the tablet is a monolayer tablet. In one embodiment, the tablet is substantially free of rilpivirine or a pharmaceutically acceptable salt thereof (e.g. the tablet contains less than 1% by weight rilpivirine or a pharmaceutically acceptable salt thereof). In one embodiment, the tablet does not contain lactose and/or starch.

The tablets disclosed herein are typically immediate release tablets. In one embodiment, the invention provides a tablet which releases at least 80% of (a) tenofovir alafenamide and/or (b) emtricitabine in 20 minutes, measured using USP apparatus II, in 500 ml of 50 mM sodium citrate pH 5.5, at 37 °C and paddle speed of 75 rpm. Typically, the tablets disclosed herein release at least 90% of (a) tenofovir alafenamide and/or (b) emtricitabine in 20 minutes, measured using USP apparatus II, in 500 ml of 50 mM sodium citrate pH 5.5, at 37 °C and paddle speed of 75 rpm.

Tablets disclosed herein will generally have a hardness within the range 13-19 kP (kilopond), and, in certain specific embodiments, have a hardness of 16 kP. Hardness can conveniently be assessed by driving an anvil to compress a tablet at a constant loading rate until it fractures, operating in accordance with USP <1217> (using *e.g.* a TBH 220, ERWEKA GmbH, Heusenstamm Germany hardness tester).

Tablets of the invention will generally have a friability of <1% by weight. Friability can be assessed according to USP <1216>.

The core of a tablet provided herein may have a hardness of between 13-19 kP, and a friability of <1% by weight.

Tablets will typically include one or more excipients. Excipients should be compatible with the other ingredients of the formulation and physiologically innocuous to the recipient thereof. Examples of suitable excipients are well known to the person skilled in the art of tablet formulation and may be found *e.g*. in Handbook of Pharmaceutical Excipients (eds. Rowe, Sheskey & Quinn), 6th edition 2009**.** As used herein the term "excipients" is intended to refer to *inter alia* basifying agents, solubilisers, glidants, fillers, binders, lubricant, diluents, preservatives, surface active agents, dispersing agents and the like. The term also includes agents such as sweetening agents, flavoring agents, coloring agents and preserving agents. Such components will generally be present in admixture within the tablet.

Examples of solubilisers include, but are not limited to, ionic surfactants (including both ionic and nonionic surfactants) such as sodium lauryl sulphate, cetyltrimethylammonium bromide, polysorbates (such as polysorbate 20 or 80), poloxamers (such as poloxamer 188 or 207), and macrogols.

Examples of lubricants, glidants and flow aids include, but are not limited to, magnesium stearate, calcium stearate, stearic acid, hydrogenated vegetable oil, glyceryl palmitostearate, glyceryl behenate, sodium stearyl fumarate, colloidal silicon dioxide, and talc. The amount of lubricant in a tablet can generally be between about 0.5-5% by weight. In certain specific embodiments, tablets of the invention include magnesium stearate. In certain embodiments, the tablet includes less than about 20 mg magnesium stearate.

Examples of disintegrants include, but are not limited to, starches, celluloses, cross-linked PVP, sodium starch glycolate, croscarmellose sodium, *etc.*

Examples of fillers (also known as bulking agents or diluents) include, but are not limited to, starches, maltodextrins, polyols (such as lactose), and celluloses. Tablets provided herein may include lactose and/or microcrystalline cellulose. Lactose can be used in anhydrous or hydrated form (e.g. monohydrate), and is typically prepared by spray drying, fluid bed granulation, or roller drying. In certain embodiments, tablets provided herein include less than about 250 mg lactose, in particular less than about 200 mg lactose, and/or less than about 250 mg microcrystalline cellulose, in particular less than about 200 mg microcrystalline cellulose. Lactose monohydrate is preferred.

Examples of binders include, but are not limited to, cross-linked PVP, HPMC, microcrystalline cellulose, sucrose, starches, *etc.*

Tablets provided herein may be uncoated or coated (in which case they include a coating). Although uncoated tablets may be used, it is more usual to provide a coated tablet, in which case a conventional non-enteric coating may be used. Film coatings are known in the art and can be composed of hydrophilic polymer materials, but are not limited to, polysaccharide materials, such as hydroxypropylmethyl cellulose (HPMC), methylcellulose, hydroxyethyl cellulose (HEC), hydroxypropyl cellulose (HPC), poly(vinylalcohol-co-ethylene glycol) and other water soluble polymers. Though the water soluble material included in the film coating of the present invention may include a single polymer material, it may also be formed using a mixture of more than one polymer. The coating may be white or coloured e.g. gray. Suitable coatings include, but are not limited to, polymeric film coatings such as those comprising polyvinyl alcohol e.g. 'Opadry^{®} II' (which includes part-hydrolysed PVA, titanium dioxide, macrogol 3350 and talc, with optional colouring such as iron oxide or indigo carmine or iron oxide yellow or FD&C yellow #6). The amount of coating will generally be between about 2-4% of the core's weight, and in certain specific embodiments, about 3%. Unless specifically stated otherwise, where the dosage form is coated, it is to be understood that a reference to % weight of the tablet means that of the total tablet, *i.e.* including the coating.

### Pharmacokinetics

### Cₘₐₓ

As used herein, Cₘₐₓ is the maximum observed plasma/serum concentration of drug.

In certain specific embodiments, tablets of the invention provide a plasma Cₘₐₓ of emtricitabine in fed patients of from about 1250 to about 2050 ng/mL, *e.g*. about 1600 ng/mL.

In certain specific embodiments, tablets of the invention provide a plasma Cₘₐₓ of tenofovir alafenamide in fed patients of from about 150 to about 260 ng/mL, *e.g*. about 200 ng/mL.

### AUC_{inf}

As used herein, AUC_{inf} is the area under the plasma/serum concentration versus time curve extrapolated to infinite time, calculated as AUC₀₋ₗₐₛₜ + (Cₗₐₛₜ/λ_{z}).

In certain specific embodiments, tablets of the invention provide a plasma AUC_{inf} of emtricitabine in fed patients of from about 7650 to about 12050 h•ng/mL, *e.g*. about 9600 h•ng/mL.

In certain specific embodiments, tablets of the invention provide a plasma AUC_{inf} of tenofovir alafenamide in fed patients of from about 200 and 340 h•ng/mL, *e.g*. about 260 h•ng/mL.

### AUCₗₐₛₜ

As used herein, AUCₗₐₛₜ is the area under the plasma/serum concentration versus time curve from time zero to the last quantifiable concentration.

In certain specific embodiments, tablets of the invention provide a plasma AUCₗₐₛₜ of emtricitabine in fed patients of from about 7500 to about 12000 h•ng/mL, e.g. about 9400 h•ng/mL.

In certain specific embodiments, tablets of the invention provide a plasma AUCₗₐₛₜ of tenofovir alafenamide in fed patients of from about 200 and 315 h•ng/mL, e.g. about 250 h•ng/mL.

### Cₗₐₛₜ

As used herein, Cₗₐₛₜ is the last observed quantifiable plasma/serum concentration of the drug.

Cₘₐₓ, Cₗₐₛₜ, AUC_{inf}, and AUCₗₐₛₜ are standard pharmacokinetic parameters that can be estimated manually or by using modelling software well known in the art, such as the Pharsight WinNonlin package using a non-compartmental model. The general basis for calculation of these quantities is well-known (*e.g*. see Rowland & Tozer (2010) Clinical Pharmacokinetics and Pharmacodynamics: Concepts and Applications ISBN 978-0781750097, or Jambhekar & Breen (2012) Basic Pharmacokinetics ISBN 978-0853699804). Typically the parameters will be assessed as the average (e.g. geometric or arithmetic mean) from within a group of at least 12 (and normally between 24 and 36) healthy human adults. Parameters should be measured in accordance with standards and practices which would be acceptable to a pharmaceutical regulatory agency such as FDA, EMA, MHLW, or WHO. The values may be based on measurements taken at appropriate intervals following the time of tablet ingestion, such as every hour, or at increasingly sparse sampling intervals, such as 1, 3, 5, 7, 9, 11, 13, 15, 20, and 24 hours after ingestion. They can be assessed either following a single-dose of drug or at steady state, but will typically be assessed following a single-dose.

The inventors have also found that it is possible to formulate emtricitabine and tenofovir alafenamide (in particular tenofovir alafenamide hemifumarate) in a tablet which is capable of demonstrating bioequivalence, *i.e*. equivalent systemic exposure (AUC_{inf}, Cₘₐₓ), for each active ingredient compared to standard comparators. In particular, some embodiments the tablets of the invention provide plasma concentrations (AUC_{inf}, Cₘₐₓ) of one or more of the active pharmaceutical ingredients that are bioequivalent to the plasma concentrations produced by the administration of a fixed dose combination of elvitegravir, cobicistat, emtricitabine, and tenofovir alafenamide hemifumarate (E/C/F/TAF) (corresponding to 150/150/200/10 mg of free base), the latter of which is the subject of a New Drug Application filed in November 2014 with the U.S. Food and Drug Administration. In some embodiments the tablets of the invention, when co-administered with elvitegravir (EVG) 150 mg tablets and cobicistat (COBI) 150 mg tablets, provide plasma concentrations (AUC_{inf}, Cₘₐₓ) of one or more of the active pharmaceutical ingredients that are bioequivalent to the plasma concentrations produced by the administration of a fixed dose combination of elvitegravir, cobicistat, emtricitabine, and tenofovir alafenamide hemifumarate (E/C/F/TAF) (corresponding to 150/150/200/10 mg of free base). In some embodiments the tablets of the invention provide plasma concentrations (AUC_{inf}, Cₘₐₓ) of one or more of the active pharmaceutical ingredients that are bioequivalent to the plasma concentrations produced by the co-administration of Emtriva^{®} (emtricitabine) 200 mg capsules and tenofovir alafenamide hemifumarate (TAF) 25 mg single-agent tablets.

Based on the findings of the inventors and the present disclosure, the skilled person is able to provide tablets which provide such bioequivalence (see for instance the examples, below).

Accordingly, in one embodiment a tablet is provided as described herein, wherein the tablet:
(a) releases emtricitabine *in vivo* in fed human subjects to provide a plasma Cₘₐₓ of from about 1300 to about 2100 ng/mL and/or a AUC_{inf} of from about 8200 to about 13200 h•ng/mL, and/or
(b) releases tenofovir alafenamide *in vivo* in fed human subjects to provide a plasma Cₘₐₓ of from about 240 to about 390 ng/mL and/or a AUC_{inf} of from about 280 and 450 h•ng/mL.

In one embodiment a tablet is provided as described herein, wherein the tablet:
(a) releases emtricitabine *in vivo* in fed human subjects to provide a plasma Cₘₐₓ of from about 1250 to about 2050 ng/mL and/or a AUC_{inf} of from about 8500 to about 13400 h•ng/mL, and/or
(b) releases tenofovir alafenamide *in vivo* in fed human subjects to provide a plasma Cₘₐₓ of from about 200 to about 360 ng/mL and/or a AUC_{inf} of from about 300 and 500 h•ng/mL.

In one embodiment a tablet is provided as described herein, wherein the tablet:
(a) releases emtricitabine *in vivo* in fed human subjects to provide a plasma Cₘₐₓ of from about 1400 to about 2300 ng/mL and/or a AUC_{inf} of from about 7400 to about 11600 h•ng/mL, and/or
(b) releases tenofovir alafenamide *in vivo* in fed human subjects to provide a plasma Cₘₐₓ of from about 160 to about 270 ng/mL and/or a AUC_{inf} of from about 190 and 320 h•ng/mL.

In some embodiments, a tablet as described herein is provided, for which the 90% confidence intervals of log-transformed Cₘₐₓ and log-transformed AUC_{inf} for emtricitabine and tenofovir alafenamide hemifumarate in fed human subjects fall completely within the range 80-125% of the log-transformed Cₘₐₓ and log-transformed AUC_{inf}, respectively, of a reference tablet, wherein the reference tablet has (i) a core consisting of 150 mg elvitegravir, 60.8 mg lactose monohydrate, 241.5 mg microcrystalline cellulose, 7.5 mg hydroxypropyl cellulose, 11.3 mg sodium lauryl sulfate, 65.8 mg croscarmellose sodium, 200 mg emtricitabine, 11.2 mg tenofovir alafenamide hemifumarate, 288.5 mg cobicistat on silicon dioxide (corresponding to 150 mg of cobicistat), 13.5 mg magnesium stearate, and (ii) a film coating consisting of 31.5 mg of a mixture of polyvinyl alcohol, titanium dioxide, polyethylene glycol, talc, indigo carmine and iron oxide (such as Opadry^{®} II Green).

In some embodiments, a tablet as described herein is provided, for which, when co-administered with elvitegravir (EVG) 150 mg tablets and cobicistat (COBI) 150 mg tablets, the 90% confidence intervals of log-transformed Cₘₐₓ and log-transformed AUC_{inf} for emtricitabine and tenofovir alafenamide hemifumarate in fed human subjects fall completely within the range 80-125% of the log-transformed Cₘₐₓ and log-transformed AUC_{inf}, respectively, of a reference tablet, wherein the reference tablet has (i) a core consisting of 150 mg elvitegravir, 60.8 mg lactose monohydrate, 241.5 mg microcrystalline cellulose, 7.5 mg hydroxypropyl cellulose, 11.3 mg sodium lauryl sulfate, 65.8 mg croscarmellose sodium, 200 mg emtricitabine, 11.2 mg tenofovir alafenamide hemifumarate, 288.5 mg cobicistat on silicon dioxide (corresponding to 150 mg of cobicistat), 13.5 mg magnesium stearate, and (ii) a film coating consisting of 31.5 mg of a mixture of polyvinyl alcohol, titanium dioxide, polyethylene glycol, talc, indigo carmine and iron oxide (such as Opadry^{®} II Green).

In some embodiments, a tablet as described herein is provided, for which the 90% confidence intervals of log-transformed Cₘₐₓ and log-transformed AUC_{inf} for emtricitabine and tenofovir alafenamide hemifumarate in fed human subjects fall completely within the range 80-125% of the log-transformed Cₘₐₓ and log-transformed AUC_{inf}, respectively, of a reference therapy, wherein the reference therapy consists of administration of (i) a tenofovir alafenamide hemifumarate tablet, consisting of 25 mg tenofovir alafenamide (as the hemifumarate salt), lactose monohydrate, microcrystalline cellulose, croscarmellose sodium, magnesium stearate, wherein the tablets are film coated with polyvinyl alcohol, titanium dioxide, polyethylene glycol, talc and yellow iron oxide, and (ii) an emtricitabine hard gelatin capsule, consisting of 200 mg emtricitabine, crospovidone, magnesium stearate, microcrystalline cellulose, povidone, titanium dioxide, gelatin and blue #2/indigo carmine aluminium lake.

### Cₘₐₓ

In certain specific embodiments, tablets of the invention provide a plasma Cₘₐₓ of emtricitabine in fed patients of from about 1300 to about 2100 ng/mL, *e.g*. about 1660 ng/mL.

In certain specific embodiments, tabletsof the invention provide a plasma Cₘₐₓ of emtricitabine in fed patients of from about 1250 to about 2050 ng/mL, *e.g*. about 1600 ng/mL.

In certain specific embodiments, tabletsof the invention provide a plasma Cₘₐₓ of emtricitabine in fed patients of from about 1400 to about 2300 ng/mL, *e.g*. about 1800 ng/mL.

In certain specific embodiments, tabletsof the invention provide a plasma Cₘₐₓ of tenofovir alafenamide in fed patients of from about 240 to about 390 ng/mL, *e.g*. about 300 ng/mL.

In certain specific embodiments, tabletsof the invention provide a plasma Cₘₐₓ of tenofovir alafenamide in fed patients of from about 200 to about 360 ng/mL, *e.g*. about 280 ng/mL.

In certain specific embodiments, tabletsof the invention provide a plasma Cₘₐₓ of tenofovir alafenamide in fed patients of from about 160 to about 270 ng/mL, *e.g*. about 200 ng/mL.

### AUC_{inf}

In certain specific embodiments, tabletsof the invention provide a plasma AUC_{inf} of emtricitabine in fed patients of from about 8200 to about 13200 h•ng/mL, *e.g*. about 10500 h•ng/mL.

In certain specific embodiments, tabletsof the invention provide a plasma AUC_{inf} of emtricitabine in fed patients of from about 8500 to about 13400 h•ng/mL, *e.g*. about 9700 h•ng/mL.

In certain specific embodiments, tabletsof the invention provide a plasma AUC_{inf} of emtricitabine in fed patients of from about 7400 to about 11600 h•ng/mL, *e.g*. about 9300 h•ng/mL.

In certain specific embodiments, tabletsof the invention provide a plasma AUC_{inf} of tenofovir alafenamide in fed patients of from about 280 and 450 h•ng/mL, *e.g*. about 350 h•ng/mL.

In certain specific embodiments, tabletsof the invention provide a plasma AUC_{inf} of tenofovir alafenamide in fed patients of from about 300 and 500 h•ng/mL, *e.g*. about 400 h•ng/mL.

In certain specific embodiments, tabletsof the invention provide a plasma AUC_{inf} of tenofovir alafenamide in fed patients of from about 190 and 320 h•ng/mL, *e.g*. about 250 h•ng/mL.

### AUCₗₐₛₜ

In certain specific embodiments, tabletsof the invention provide a plasma AUCₗₐₛₜ of emtricitabine in fed patients of from about 8000 to about 13000 h•ng/mL, *e.g*. about 10200 h•ng/mL.

In certain specific embodiments, tabletsof the invention provide a plasma AUCₗₐₛₜ of emtricitabine in fed patients of from about 7500 to about 11800 h•ng/mL, *e.g*. about 9400 h•ng/mL.

In certain specific embodiments, tabletsof the invention provide a plasma AUCₗₐₛₜ of emtricitabine in fed patients of from about 7200 to about 11400 h•ng/mL, *e.g*. about 9000 h•ng/mL.

In certain specific embodiments, tabletsof the invention provide a plasma AUCₗₐₛₜ of tenofovir alafenamide in fed patients of from about 260 and 430 h•ng/mL, *e.g*. about 340 h•ng/mL.

In certain specific embodiments, tabletsof the invention provide a plasma AUCₗₐₛₜ of tenofovir alafenamide in fed patients of from about 280 and 470 h•ng/mL, *e.g*. about 370 h•ng/mL.

In certain specific embodiments, tabletsof the invention provide a plasma AUCₗₐₛₜ of tenofovir alafenamide in fed patients of from about 170 and 310 h•ng/mL, *e.g*. about 250 h•ng/mL.

### Cₗₐₛₜ

It is well known in the bioavailability and bioequivalence arts how to determine whether any particular tablet meets regulatory requirements for equivalent bioavailability and pharmacokinetic bioequivalence *e.g.* see: Niazi (2014) Handbook of Bioequivalence Testing, 2nd Edition, ISBN 978-1482226379; Guidance for Industry Bioavailability and Bioequivalence Studies for Orally Administered Drug Products - General Considerations FDA March 2003; and Guideline On The Investigation Of Bioequivalence, EMEA 2010 CPMP/EWP/QWP/1401/98 Rev. 1/ Corr **. To ensure statistical power a study to measure the Cₘₐₓ, AUCₗₐₛₜ and AUC_{inf} values will be performed in multiple subjects *e.g.* in a group of at least 12 (and normally between 24 and 36) healthy human adults.

Because determining the Cₘₐₓ, AUCₗₐₛₜ and AUC_{inf} values is necessarily destructive these parameters will not be determined directly for the dosage form (in particular the tablet) in question, but rather for a dosage form made by the same manufacturing process with the same components. Thus a batch of a dosage form (e.g. tablets) can be made by a particular process, and the 90% confidence interval of Cₘₐₓ, AUCₗₐₛₜ and AUC_{inf} will be assessed on a sample of those tablets. If these values meet the 80-125% requirement noted above then tablets made by the manufacturing process in question are tablets of the present invention.

### Stability

As mentioned above and as explained in more detail in the examples below, the stability of tenofovir alafenamide deteriorates in the presence of emtricitabine. The degradation of tenofovir alafenamide is further accelerated in the presence of rilpivirine. Known degradation products of tenofovir alafenamide include PMPA and PMPA anhydride. Similarly, the stability of emtricitabine in the presence of tenofovir alafenamide is a challenge in formulating a composition comprising these three active ingredients. Known degradation products of emtricitabine include cyclic-FTU-1 and FTU.

The inventors have found that tabletsof the invention are stable, *i.e.* they have acceptable shelf-life, despite the dosage forms containing tenofovir alafenamide and emtricitabine. Accordingly, tabletsthat do not comprise a pharmaceutically unacceptable amount of a tenofovir alafenamide degradation product are provided. Also provided is a tablet comprising (a) tenofovir alafenamide or a pharmaceutically acceptable salt thereof in the amount specified in claim 1, and (b) emtricitabine or a pharmaceutically acceptable salt thereof in the amount specified in claim 1, where the total quantity of degradation products derived from the tenofovir alafenamide or the pharmaceutically acceptable salt thereof is less than 5% (for instance less than about 4.2%, such as less than 4%) after storage for six months at 40°C/75% RH in closed conditions. Also provided is a tablet comprising (a) tenofovir alafenamide or a pharmaceutically acceptable salt thereof in the amount specified in claim 1, and (b) emtricitabine or a pharmaceutically acceptable salt thereof in the amount specified in claim 1, where the total quantity of degradation products derived from the tenofovir alafenamide or the pharmaceutically acceptable salt thereof is less than 2.5% (such as less than 2%, for instance about 1.7% or about 1.8%) after storage for three months at 40°C/75% RH in closed conditions. Also provided is a tablet comprising (a) tenofovir alafenamide or a pharmaceutically acceptable salt thereof in the amount specified in claim 1, and (b) emtricitabine or a pharmaceutically acceptable salt thereof in the amount specified in claim 1, where the total quantity of degradation products derived from the tenofovir alafenamide or the pharmaceutically acceptable salt thereof is less than 3% (such as less than 2%) after storage for one month at 40°C/75% RH in open conditions. In another embodiment, a kit comprising (a) the tablet according to the claims comprising tenofovir alafenamide or a pharmaceutically acceptable salt thereof, and emtricitabine or a pharmaceutically acceptable salt thereof, and (b) a desiccant (e.g. silica gel) is provided.

The inventors have conducted drug load studies and have realized that the chemical stability of tenofovir alafenamide varies depending on the proportion of tenofovir alafenamide within a given composition. Accordingly, a tablet comprising tenofovir alafenamide or a pharmaceutically acceptable salt thereof is provided, wherein the proportion of tenofovir alafenamide in the tablet is from 4% to 9% by weight. In some embodiments, a tablet is provided which comprises from 7% to 9% by weight tenofovir alafenamide hemifumarate, in particular about 8% by weight tenofovir alafenamide hemifumarate.

In addition, tenofovir alafenamide undergoes solid-state hydrolysis and accordingly the inclusion of desiccant may assist in facilitating an acceptable shelf-life. Thus, a kit is provided which comprises (a) a tablet according to the claims comprising tenofovir alafenamide or a pharmaceutically acceptable salt thereof, and emtricitabine or a pharmaceutically acceptable salt thereof, and (b) a desiccant. The inventors have observed that the stability of tenofovir alafenamide in certain formulations is dependent on desiccant level. In certain embodiments, therefore, the kit includes silica gel as a desiccant. In certain specific embodiments, the kit includes 3 g silica gel as a desiccant. The kit may optionally further include polyester coil packing material. In certain embodiments, the total quantity of degradation products derived from the tenofovir alafenamide or the pharmaceutically acceptable salt thereof in the tablet is less than about 2%, e.g., less than about 1.5% or less than about 1%, after storage for 12 months at 30°C/75% RH. In certain embodiments, the total quantity of degradation products derived from the tenofovir alafenamide or the pharmaceutically acceptable salt thereof is less than 2.5% (such as less than 2%, for instance about 1.7% or about 1.8%) after storage for three months at 40°C/75% RH. The kit of the invention may comprise a bottle suitable for storing the oral dosage forms described herein.

The invention provides a tablet according to the appended claims comprising tenofovir alafenamide or a pharmaceutically acceptable salt thereof and emtricitabine or a pharmaceutically acceptable salt thereof. In one embodiment, this tablet is coated. In one embodiment, this coating is a film coating, such as Opadry II.

In one embodiment, the tablet comprises microcrystalline cellulose and croscarmellose sodium.

In one embodiment, the tablet comprises microcrystalline cellulose, croscarmellose sodium and magnesium stearate.

In one embodiment, the tablet comprises 20-30 mg of croscarmellose sodium. In one embodiment, the tablet comprises 80-90 mg of microcrystalline sodium. In one embodiment, the tablet comprises 2-7 mg of magnesium stearate.

In one embodiment, the tablet does not comprise lactose. In one embodiment, the tablet does not comprise starch. In one embodiment, the tablet comprises neither lactose nor starch.

In one embodiment, the tablet has a total weight of less than 600 mg, or less than 500 mg, or less than 400 mg, or less than 375 mg. In one embodiment, the tablet has a total weight of 350 mg ± 50 mg or 350 mg ± 25 mg, or 350 mg ± 5 mg, or 350 mg.

In one embodiment the tablet is coated and the tablet has a total weight of less than 600 mg, such as less than 500 mg, less than 400 mg, or less than 375 mg. In one embodiment the tablet is coated and the tablet has a total weight of 360.5 mg ± 50 mg, such as 360.5 mg ± 25 mg, 360.5 mg ± 5 mg, or 360.5 mg.

In one embodiment, over 40 % by weight of the tablet is emtricitabine or a salt thereof and tenofovir alafenamide or a salt thereof. In one embodiment, over 50 % by weight of the tablet is emtricitabine or a salt thereof and tenofovir alafenamide or a salt thereof. In one embodiment, over 60 % by weight of the tablet of the invention is emtricitabine or a salt thereof and tenofovir alafenamide or a salt thereof. In one embodiment, over 64 % by weight of the tablet is emtricitabine or a salt thereof and tenofovir alafenamide or a salt thereof. In one embodiment, over 65 % by weight of the tablet is emtricitabine or a salt thereof and tenofovir alafenamide or a salt thereof. In one embodiment of the invention, over 65 % by weight of the tablet is emtricitabine and tenofovir alafenamide hemifumarate.

In one embodiment, about 40 % to about 75% by weight of the tablet is emtricitabine or a salt thereof and tenofovir alafenamide or a salt thereof. In one embodiment, about 50 % to about 75% by weight of the tablet is emtricitabine or a salt thereof and tenofovir alafenamide or a salt thereof. In one embodiment, about 50 % to about 70% by weight of the tablet is emtricitabine or a salt thereof and tenofovir alafenamide or a salt thereof. In one embodiment, about 60 % to about 75% by weight of the tablet of the invention is emtricitabine or a salt thereof and tenofovir alafenamide or a salt thereof. In one embodiment, about 64 % to about 75% by weight of the tablet is emtricitabine or a salt thereof and tenofovir alafenamide or a salt thereof. In one embodiment, about 65 % to about 75% by weight of the tablet is emtricitabine or a salt thereof and tenofovir alafenamide or a salt thereof.

In one embodiment, the tablet is coated and over 40 % by weight of the tablet is emtricitabine or a pharmaceutically acceptable salt thereof and tenofovir alafenamide or a pharmaceutically acceptable salt thereof. In one embodiment, the tablet is coated and over 50 % by weight of the tablet is emtricitabine or a pharmaceutically acceptable salt thereof and tenofovir alafenamide or a pharmaceutically acceptable salt thereof. In one embodiment, the tablet is coated and over 58 % by weight of the tablet of the invention is emtricitabine or a pharmaceutically acceptable salt thereof and tenofovir alafenamide or a pharmaceutically acceptable salt thereof. In one embodiment, the tablet is coated and over 60 % by weight of the tablet is emtricitabine or a pharmaceutically acceptable salt thereof and tenofovir alafenamide or a pharmaceutically acceptable salt thereof. In one embodiment, the tablet is coated and over 63 % by weight of the tablet is emtricitabine or a pharmaceutically acceptable salt thereof and tenofovir alafenamide or a pharmaceutically acceptable salt thereof.

In one embodiment, the tablet is coated and about 40 % to about 75% by weight of the tablet is emtricitabine or a pharmaceutically acceptable salt thereof and tenofovir alafenamide or a pharmaceutically acceptable salt thereof. In one embodiment, the tablet is coated and about 50 % to about 75% by weight of the tablet is emtricitabine or a pharmaceutically acceptable salt thereof and tenofovir alafenamide or a pharmaceutically acceptable salt thereof. In one embodiment, the tablet is coated and about 50 % to about 70% by weight of the tablet is emtricitabine or a pharmaceutically acceptable salt thereof and tenofovir alafenamide or a pharmaceutically acceptable salt thereof. In one embodiment, the tablet is coated and about 58 % to about 70% by weight of the tablet of the invention is emtricitabine or a pharmaceutically acceptable salt thereof and tenofovir alafenamide or a pharmaceutically acceptable salt thereof. In one embodiment, the tablet is coated and about 60 % to about 70% by weight of the tablet is emtricitabine or a pharmaceutically acceptable salt thereof and tenofovir alafenamide or a pharmaceutically acceptable salt thereof. In one embodiment, the tablet is coated and about 63 % to about 70% by weight of the tablet is emtricitabine or a pharmaceutically acceptable salt thereof and tenofovir alafenamide or a pharmaceutically acceptable salt thereof.

In one embodiment of the invention, the tablet is coated and over 50 % by weight of the tablet is emtricitabine and tenofovir alafenamide hemifumarate. In one embodiment of the invention, the tablet is coated and over 58 % by weight of the tablet is emtricitabine and tenofovir alafenamide hemifumarate. In one embodiment of the invention, the tablet is coated and over 63 % by weight of the tablet is emtricitabine and tenofovir alafenamide hemifumarate.

In one embodiment of the invention, the tablet is coated and about 50 % to about 75% by weight of the tablet is emtricitabine and tenofovir alafenamide hemifumarate. In one embodiment of the invention, the tablet is coated and about 58 % to about 70% by weight of the tablet is emtricitabine and tenofovir alafenamide hemifumarate. In one embodiment of the invention, the tablet is coated and about 63 % to about 70% by weight of the tablet is emtricitabine and tenofovir alafenamide hemifumarate.

In one embodiment of the invention, the tablet contains less than 250 mg of excipients, for example less than 200 mg, or less than 150 mg, or less than 130 mg, or less than 120 mg of excipients.

In one embodiment of the invention, at least 50% by weight of the tablet is emtricitabine. In one embodiment of the invention, at least 55% by weight of the tablet is emtricitabine.

In one embodiment of the invention, about 50% to about 65% by weight of the tablet is emtricitabine. In one embodiment of the invention, about 55% to about 60% by weight of the tablet is emtricitabine.

In another embodiment, the tablet comprises 7-9% by weight tenofovir alafenamide hemifumarate, preferably about 8% by weight tenofovir alafenamide hemifumarate.

In one embodiment of the invention, the tablet comprises a) tenofovir alafenamide or a pharmaceutically acceptable salt thereof in the amount specified in claim 1 and b) emtricitabine or a pharmaceutically acceptable salt thereof in the amount specified in claim 1, and the total quantity of degradation products derived from the tenofovir alafenamide or a pharmaceutically acceptable salt thereof is less than about 2.5%, such as less than 2%, for instance about 1.7% or 1.8%, after storage for 3 months at 40°C/75% RH in closed conditions.

In one embodiment of the invention, the tablet comprises a) tenofovir alafenamide hemifumarate in the amount specified in claim 1 and b) emtricitabine in the amount specified in claim 1, and the total quantity of degradation products derived from the tenofovir alafenamide hemifumarate is less than about 2.5%, such as less than 2%, for instance about 1.7% or 1.8%, after storage for 3 months at 40°C/75% RH in closed conditions.

In one embodiment of the invention, the tablet comprises 7-9% by weight tenofovir alafenamide or a pharmaceutically acceptable salt thereof, preferably about 8% by weight tenofovir alafenamide or a pharmaceutically acceptable salt thereof, and the total quantity of degradation products derived from the tenofovir alafenamide or a pharmaceutically acceptable salt thereof is less than about 2%, for instance about 1.7%, after storage for 3 months at 40°C/75% RH in closed conditions.

In one embodiment of the invention, the tablet comprises 7-9% by weight tenofovir alafenamide hemifumarate, preferably about 8% by weight tenofovir alafenamide hemifumarate, and the total quantity of degradation products derived from the tenofovir alafenamide hemifumarate is less than about 2%, for instance about 1.7%, after storage for 3 months at 40°C/75% RH in closed conditions.

In another embodiment of the invention, the tablet comprises about 8% by weight tenofovir alafenamide hemifumarate and at least 55% by weight emtricitabine, wherein the total quantity of degradation products derived from the tenofovir alafenamide hemifumarate is less than about 2%, for instance about 1.7%, after storage for 3 months at 40°C/75% RH in closed conditions.

In one embodiment of the invention, the tablet comprises a) tenofovir alafenamide hemifumarate in the amount specified in claim 1 and b) emtricitabine in the amount specified in claim 1, and the total quantity of degradation products derived from the tenofovir alafenamide hemifumarate is less than about 2%, such as less than 1.5% or less than 1%, after storage for 12 months at 30°C/75% RH in closed conditions.

In one embodiment of the invention, the tablet comprises 7-9% by weight tenofovir alafenamide or a pharmaceutically acceptable salt thereof, preferably about 8% by weight tenofovir alafenamide or a pharmaceutically acceptable salt thereof, and the total quantity of degradation products derived from the tenofovir alafenamide or a pharmaceutically acceptable salt thereof is less than about 2%, such as less than 1.5% or less than 1%, after storage for 12 months at 30°C/75% RH in closed conditions.

In one embodiment of the invention, the tablet comprises 7-9% by weight tenofovir alafenamide hemifumarate, preferably about 8% by weight tenofovir alafenamide hemifumarate, and the total quantity of degradation products derived from the tenofovir alafenamide hemifumarate is less than about 2%, such as less than 1.5% or less than 1%, after storage for 12 months at 30°C/75% RH in closed conditions.

In another embodiment of the invention, the tablet comprises about 8% by weight tenofovir alafenamide hemifumarate and at least 55% by weight emtricitabine, wherein the total quantity of degradation products derived from the tenofovir alafenamide hemifumarate is less than about 2%, such as less than 1.5% or less than 1%, after storage for 12 months at 30°C/75% RH in closed conditions.

In one embodiment of the invention, less than 20% by weight of the tablet is croscarmellose sodium. In one embodiment of the invention, less than 10% by weight of the tablet is croscarmellose sodium.

In one embodiment of the invention, less than 40% by weight of the tablet is microcrystalline cellulose. In one embodiment of the invention, less than 30% by weight of the tablet is microcrystalline cellulose. In one embodiment of the invention, less than 26% by weight of the tablet is microcrystalline cellulose.

In one embodiment of the invention, the tablet comprises 200 mg emtricitabine and 28 mg tenofovir alafenamide hemifumarate, wherein at least 50% of the total weight of the tablet is emtricitabine and tenofovir alafenamide hemifumarate. In one embodiment of the invention, the tablet comprises 200 mg emtricitabine and 28 mg tenofovir alafenamide hemifumarate, wherein at least 50% of the total weight of the tablet is emtricitabine and tenofovir alafenamide hemifumarate, and wherein the excipients consist of croscarmellose sodium, microcrystalline cellulose and magnesium stearate. In one embodiment of the invention, this tablet is coated with a film coating, optionally wherein the film coating is Opadry^{®} II and/or the amount of the coating is 2-4% of the weight of the core of the tablet. In one embodiment of the invention, the total weight of this tablet is 350 mg. In one embodiment of the invention, this tablet is coated and the total weight of this tablet is 360.5 mg.

In one embodiment of the invention, the tablet comprises 200 mg emtricitabine and 28 mg tenofovir alafenamide hemifumarate, wherein at least 60% of the total weight of the tablet is emtricitabine and tenofovir alafenamide hemifumarate. In one embodiment of the invention, the tablet comprises 200 mg emtricitabine and 28 mg tenofovir alafenamide hemifumarate, wherein at least 60% of the total weight of the tablet is emtricitabine and tenofovir alafenamide hemifumarate, and wherein the excipients consist of croscarmellose sodium, microcrystalline cellulose and magnesium stearate. In one embodiment of the invention, this tablet is coated with a film coating, optionally wherein the film coating is Opadry^{®} II and/or the amount of the coating is 2-4% of the weight of the core of the tablet. In one embodiment of the invention, the total weight of this tablet is 350 mg. In one embodiment of the invention, this tablet is coated and the total weight of this tablet is 360.5 mg.

In one embodiment of the invention, the tablet comprises 200 mg emtricitabine and 28 mg tenofovir alafenamide hemifumarate, wherein at least 63% of the total weight of the tablet is emtricitabine and tenofovir alafenamide hemifumarate. In one embodiment of the invention, the tablet comprises 200 mg emtricitabine and 28 mg tenofovir alafenamide hemifumarate, wherein at least 63% of the total weight of the tablet is emtricitabine and tenofovir alafenamide hemifumarate, and wherein the excipients consist of croscarmellose sodium, microcrystalline cellulose and magnesium stearate. In one embodiment of the invention, this tablet is coated with a film coating, optionally wherein the film coating is Opadry^{®} II and/or the amount of the coating is 2-4% of the weight of the core of the tablet. In one embodiment of the invention, the total weight of this tablet is 350 mg. In one embodiment of the invention, this tablet is coated and the total weight of this tablet is 360.5 mg.

In one embodiment of the invention, the tablet comprises 200 mg emtricitabine and 28 mg tenofovir alafenamide hemifumarate, wherein at least 65% of the total weight of the tablet is emtricitabine and tenofovir alafenamide hemifumarate. In one embodiment of the invention, the tablet comprises 200 mg emtricitabine and 28 mg tenofovir alafenamide hemifumarate, wherein at least 65% of the total weight of the tablet is emtricitabine and tenofovir alafenamide hemifumarate, and wherein the excipients consist of croscarmellose sodium, microcrystalline cellulose and magnesium stearate. In one embodiment of the invention, this tablet is coated with a film coating, optionally wherein the film coating is Opadry^{®} II and/or the amount of the coating is 2-4% of the weight of the core of the tablet. In one embodiment of the invention, the total weight of this tablet is 350 mg. In one embodiment of the invention, this tablet is coated and the total weight of this tablet is 360.5 mg.

In another embodiment of the invention, the tablets disclosed herein are part of a kit comprising (a) a tablet comprising tenofovir alafenamide or a pharmaceutically acceptable salt thereof, and emtricitabine or a pharmaceutically acceptable salt thereof, and (b) a desiccant (*e.g*. silica gel).

In one embodiment of the invention, the tablet of the invention consists of:

| **Ingredient** | **Mass (mg)** |
|---|---|
| Emtricitabine | 200 |
| Tenofovir alafenamide hemifumarate | 28 |
| Croscarmellose sodium | 28 |
| Microcrystalline cellulose | 89 |
| Magnesium stearate | 5.3 |

and optionally a film coating, for example a film coating comprising Opadry II Blue 85F105057 (a combination of Polyvinyl alcohol, polyethylene glycol (PEG), talc, titanium dioxide, FD&C blue #2).

In one embodiment of the invention, the tablet of the invention consists of:

| **Ingredient** | **Mass (mg)** |
|---|---|
| Emtricitabine | 200.00 |
| Tenofovir alafenamide hemifumarate | 28.00 |
| Croscarmellose sodium | 28.00 |
| Microcrystalline cellulose | 88.70 |
| Magnesium stearate | 5.25 |

and optionally a film coating, for example a film coating comprising Opadry II Blue 85F105057 (a combination of Polyvinyl alcohol, polyethylene glycol (PEG), talc, titanium dioxide, FD&C blue #2).

In one embodiment of the invention, the tablet of the invention consists of:

| **Ingredient** | **Mass (mg)** |
|---|---|
| *Intragranular* | |
| Emtricitabine | 200.00 |
| Tenofovir alafenamide hemifumarate | 28.00 |
| Croscarmellose sodium | 28.00 |
| Microcrystalline cellulose | 88.70 |
| Magnesium stearate | 2.625 |

| *Extragranular* | |
|---|---|
| Magnesium stearate | 2.625 |
| *Total core weight* | *350* |

and a film coating consisting of Opadry II Blue 85F105057 (which contains 40.0% w/w Polyvinyl Alcohol-part hydrolyzed, 23.32% w/w Titanium Dioxide, 20.2% w/w Macrogol/PEG 3350, 14.8% w/w Talc, and 1.68% w/w FD&C Blue #2/Indigo Carmine Aluminum Lake).

In one embodiment of the invention, the tablet of the invention consists of:

| **Ingredient** | **Mass (mg)** |
|---|---|
| *Intragranular* | |
| Emtricitabine | 200 |
| Tenofovir alafenamide hemifumarate | 28 |
| Croscarmellose sodium | 28 |
| Microcrystalline cellulose | 89 |
| Magnesium stearate | 2.6 |

| *Extragranular* | |
|---|---|
| Magnesium stearate | 2.6 |
| *Total core weight* | *350* |

and a film coating consisting of Opadry II Blue 85F105057 (which contains 40.0% w/w Polyvinyl Alcohol-part hydrolyzed, 23.32% w/w Titanium Dioxide, 20.2% w/w Macrogol/PEG 3350, 14.8% w/w Talc, and 1.68% w/w FD&C Blue #2/Indigo Carmine Aluminum Lake).

### Manufacturing methods

Methods for producing the tablets described herein are also disclosed (but not claimed). In some embodiments, the method comprises blending emtricitabine and tenofovir alafenamide hemifumarate with excipients, followed by compression. In some embodiments, emtricitabine and tenofovir alafenamide hemifumarate are first co-blended and granulated with excipients, for example by dry granulation. This step involves, in certain embodiments, roller compaction and/or milling. In some embodiments, the granulation of the co-blended emtricitabine and tenofovir alafenamide hemifumarate are further combined with extragranular excipients, including but not limited to magnesium stearate, then compressed.

Typically, the methods will include a step of coating the tablet cores after compression, e.g. with a film coating as described above.

In general, tableting methods are well known in the art of pharmacy. Techniques and formulations generally are found in Remington's Pharmaceutical Sciences (Mack Publishing Co., Easton, PA).

A tablet can be made by compression or molding, optionally with one or more excipients. Compressed tablets may be prepared by compressing in a suitable machine the active ingredient in a free-flowing form such as a powder or granules, optionally mixed with excipients.

### Therapeutic methods

The tablets disclosed herein may be used to treat or prevent HIV (*e.g*. HIV-1). In some embodiments, the tablets disclosed herein may be used to treat or prevent HIV-1 or HIV-2.

In certain embodiments, the tablets disclosed herein may be used to treat HIV (*e.g.* HIV-1). In some embodiments, the tablets disclosed herein may be used to treat HIV-1 or HIV-2.

In certain embodiments, the tablets disclosed herein may be used to prevent HIV (*e.g.* HIV-1). In some embodiments, the tablets disclosed herein may be used to prevent HIV-1 or HIV-2.

Accordingly, a tablet of the invention is provided for use in methods for treating a subject having HIV, comprising administering a tablet of the invention.

In certain embodiments, a tablet herein is provided for use in a method of treating an HIV infection in a human having or at risk of having the infection, wherein the method includes administering to the human the tablets disclosed herein. Similarly, a tablet herein is provided for use in a method of preventing HIV infection in a human having or at risk of becoming infected infection is provided, wherein the method includes administering to the human the tablets disclosed herein.

In another embodiment, a tablet disclosed herein for use in therapy is provided. In particular, a tablet disclosed herein is provided for use in a method of treating the proliferation of the HIV virus, treating AIDS, or delaying the onset of AIDS or ARC symptoms in a mammal (e.g., a human, such as for example men having sex with men or transsexual women having sex with men), comprising administering to the mammal a tablet disclosed herein.

In a particular embodiment, the tablets disclosed herein are provided for use in a method of preventing HIV infection from taking hold if the individual is exposed to the virus and/or to keep the virus from establishing a permanent infection and/or to prevent the appearance of symptoms of the disease and/or to prevent the virus from reaching detectable levels in the blood, for example for pre-exposure prophylaxis (PrEP) or post-exposure prophylaxis (PEP). Accordingly, in certain embodiments, tablets for use in methods for reducing the risk of acquiring HIV (e.g., HIV-1 and/or HIV-2) are provided. For example, tablets for use in methods for reducing the risk of acquiring HIV (e.g., HIV-1 and/or HIV-2) are disclosed, comprising administration of the tablets are disclosed herein. In certain specific embodiments, tablets for use in methods for reducing the risk of acquiring HIV (e.g., HIV-1 and/or HIV-2) comprising administration of a tablet disclosed herein in combination with safer sex practices. In certain embodiments, tablets for use in methods for reducing the risk of acquiring HIV (e.g., HIV-1 and/or HIV-2) comprise administration to an individual at risk of acquiring HIV. Examples of individuals at high risk for acquiring HIV include, without limitation, an individual who is at risk of sexual transmission of HIV.

In certain embodiments, the reduction in risk of acquiring HIV is at least about 40%, 50%, 60%, 70%, 80%, 90%, or 95%. In certain embodiments, the reduction in risk of acquiring HIV is at least about 75%. In certain embodiments, the reduction in risk of acquiring HIV is about 80%, 85%, or 90%.

The tablets disclosed herein are provided for use in methods that involve administering a tablet disclosed herein to the subject, typically a human, and will generally involve repeated administrations, typically once daily. The treatment may be prophylactic or therapeutic treatment.

In certain embodiments, tablets are disclosed for use in methods that involve repeated administrations at intervals less than once daily. For example, in certain embodiments, tablets are disclosed for use in methods that involve administration of the tablets disclosed herein every other day, five times per week, four times per week, three times per week, two times per week, or one time per week.

In certain embodiments, tablets are disclosed for use in methods that involve administration prior to and/or after an event that would expose the individual to HIV or that would otherwise increase the individual's risk of acquiring HIV, e.g., as pre-exposure prophylaxis (PrEP) and/or as post-exposure prophylaxis (PEP). Examples of events that could increase an individual's risk of acquiring HIV include, without limitation, no condom use during anal intercourse with an HIV positive partner or a partner of unknown HIV status; anal intercourse with more than 3 sex partners; exchange of money, gifts, shelter or drugs for anal sex; sex with male partner and diagnosis of sexually transmitted infection; and no consistent use of condoms with sex partner known to be HIV positive.

In certain embodiments, e.g., when administered as PrEP, the tablets disclosed herein are administered 2 to 72 hours, 2 to 48 hours, 2 to 24 hours, or 2 to 12 hours prior to an event that would increase the individual's risk of acquiring HIV (e.g., prior to sex or other exposure to the HIV virus). In some embodiments, the tablets disclosed herein are administered within 72 hours, 60 hours, 48 hours, 24 hours, 12 hours, 9 hours, 6 hours, 4 hours, 3 hours, 2 hours or 1 hour prior to an event that would increase the individual's risk of acquiring HIV (e.g., prior to sex or other exposure to the HIV virus). In certain embodiments, when the tablets disclosed herein are administered prior to an event that would increase the individual's risk of acquiring HIV, they are administered daily prior to the event. In certain embodiments, when the tablets disclosed herein are administered prior to an event that would increase the individual's risk of acquiring HIV, they are administered one to three times prior to the event.

In certain embodiments, e.g., when administered as part of a PrEP regimen or as PEP, the tablets disclosed herein are administered 2 to 48 hours, 2 to 36 hours, 2 to 24 hours, or 2 to 12 hours following an event that would increase the individual's risk of acquiring HIV (e.g., following sex or other exposure to the HIV virus). In certain embodiments, e.g., when administered as PEP, the tabletsdisclosed herein are administered for 7 days, 14 days, 21 days, 28 days, 30 days, or 45 days following an event that would increase the individual risk of acquiring HIV (e.g., following sex or other exposure to the HIV virus). In certain embodiments, e.g., when administered as PEP, the tabletsdisclosed herein are administered for 30 days following an event that would increase the individual risk of acquiring HIV (e.g., following sex or other exposure to the HIV virus). In certain embodiments, the tabletsdisclosed herein are administered less than 1 hour, 2 hours, 3 hours, 4, hours, 5 hours, 6 hours, 7 hours, 8 hours, 9 hours, 12 hours, 18 hours, 24 hours, 36 hours, or 48 hours following an event that would increase the individual's risk of acquiring HIV (e.g., following sex or other exposure to the HIV virus). In certain other embodiments, the tabletsdisclosed herein are administered for 1 day, 2 days, 3, days 4 days, or 5 days following an event that would increase the individual's risk of acquiring HIV (e.g., following sex or other exposure to the HIV virus). In certain embodiments, when the tabletsdisclosed herein are administered following to an event that would increase the individual's risk of acquiring HIV, they are administered daily following to the event. In certain embodiments, when the tabletsdisclosed herein are administered following an event that would increase the individual's risk of acquiring HIV, they are administered one to three times following the event. In certain embodiments, when the tabletsdisclosed herein are administered following to an event that would increase the individual's risk of acquiring HIV, they are administered once following the event.

In certain embodiments, e.g., when administered as PrEP, the tabletsdisclosed herein are administered prior to an event that would increase the individual's risk of acquiring HIV (e.g., prior to sex) and following the event. For example, in certain embodiments, e.g., when administered as PrEP, the tabletsdisclosed herein are administered 2 to 72 hours, 2 to 48 hours, 2 to 24 hours, or 2 to 12 hours prior to an event that would increase the individual's risk of acquiring HIV (e.g., prior to sex) and 2 to 48 hours, 2 to 36 hours, 2 to 24 hours, or 2 to 12 hours following the event. For example, in some embodiments, one or more (e.g., one, two, or three) tabletsdisclosed herein are administered one to three days prior to an event that would increase the individual's risk of acquiring HIV (e.g., prior to sex) and once per day for a period of one to five days following the event. In some embodiments, one or more (e.g., one, two, or three) tabletsdisclosed herein are administered 2 to 24 hours prior to an event that would increase the individual's risk of acquiring HIV (e.g., prior to sex) and one or more times (e.g., one, two, or three times) 2 to 48 hours following the event. In some embodiments, the tabletsdisclosed herein are administered once per week, twice per week, three times per week, four times per week, or five times per week and one or more times (e.g., one, two, or three times) 2 to 48 hours following an event that would increase the individual's risk of acquiring HIV (e.g., prior to sex). In one embodiment, the tabletsdisclosed herein are administered twice per week (one composition (i.e., tablet) per day) and once (one composition) following an event that increases the individual's risk of acquiring HIV (e.g., one tablet within 24 hours of exposure, such as following sex).

### General

The term "fed" in relation to administration of a solid oral dosage form to a human subject means administration of the dosage form orally under fed conditions (moderate fat meal) e.g. administration within about 30 minutes of the human consuming a standardized meal of about 300 to 600 calories and about 10 to about 15 grams of fat.

The term "substantially free" in relation to the presence of a given component within e.g. a composition means that less than 5% by weight of the composition (e.g. less than 1% by weight of the composition) is that given component. The word "substantially" does not exclude "completely" e.g. a composition which is "substantially free" from Y may be completely free from Y. Where necessary, the word "substantially" may be omitted from the definition of the invention.

The term "segregated" as used in relation to certain components (e.g. A and B) within a tablet means that those components are physically discrete such that the presence of one component (e.g. A) does not substantially affect the stability in storage of the other component(s) (e.g. B) from which it is segregated. Typically, when components are segregated in a tablet then they will be present in separate layers in a multilayer tablet. By way of example, components A and B may be present in separate layers in a multilayer tablet, wherein (a) the layer containing component A is substantially free of component B and (b) the layer containing component B is substantially free of component A. The separate layers may be in contact with each other or may be separated e.g. by one or more additional layers.

The term "comprise" and variations thereof, such as "comprises" and "comprising", are to be construed in an open, inclusive sense, that is as "including, but not limited to".

The term "between" with reference to two values includes those two values e.g. the range "between" 10 mg and 20 mg encompasses e.g. 10, 11, 12, 13, 14, 15, 16, 17, 18, 19 and 20 mg.

As used herein the term "about" is understood by the person of skill in the art to reflect the variability in the numerical value it modifies. Values expressed herein are understood to include a range of variability. To reflect this variability, any numerical value used herein incorporates this variability. As such, numerical values used herein encompass that value stated, as well as the value as modified with "about." In certain embodiments, the term "about" in relation to a numerical value x is optional and means, for example, *x*±10%*,* x+5%, or *x*±1%.

"% w/w" means the weight of a component as a percentage of the total weight of *e.g.* a layer or dosage form in which the component is present. For example, a composition comprising "5% w/w X" refers to a composition in which the weight of component X is 5% of the total weight of the composition.

Reference throughout this specification to "one embodiment" or "an embodiment" means that a particular feature, structure or characteristic described in connection with the embodiment is included in at least one embodiment provided herein. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The term "pharmaceutically acceptable" with respect to a substance refers to that substance which is generally regarded as safe and suitable for use without undue toxicity, irritation, allergic response, and the like, commensurate with a reasonable benefit/risk ratio.

"Pharmaceutically acceptable salt" refers to a salt of a compound that is pharmaceutically acceptable and that possesses (or can be converted to a form that possesses) the desired pharmacological activity of the parent compound. Such salts include acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic acid, camphorsulfonic acid, citric acid, ethanesulfonic acid, fumaric acid, glucoheptonic acid, gluconic acid, lactic acid, maleic acid, malonic acid, mandelic acid, methanesulfonic acid, 2-napththalenesulfonic acid, oleic acid, palmitic acid, propionic acid, stearic acid, succinic acid, tartaric acid, p-toluenesulfonic acid, trimethylacetic acid, and the like, and salts formed when an acidic proton present in the parent compound is replaced by either a metal ion, e.g., an alkali metal ion, an alkaline earth ion, or an aluminum ion; or coordinates with an organic base such as diethanolamine, triethanolamine, N-methylglucamine and the like. Also included in this definition are ammonium and substituted or quatemized ammonium salts. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5.

As used herein, the term "salts" includes co-crystals. The term "co-crystal" refers to a crystalline compound comprising two or more molecular components, e.g. wherein proton transfer between the molecular components is partial or incomplete. Accordingly, the term "pharmaceutically acceptable salt" as used herein encompasses salts and co-crystals as defined herein.

The term "solvate" means a molecular complex comprising a compound and one or more pharmaceutically acceptable solvent molecules. Examples of solvent molecules include water and C₁₋₆ alcohols, e.g. ethanol. When the solvate is water, the term "hydrate" may be used.

"Treating" and "treatment" of a disease include the following:
(1) preventing or reducing the risk of developing the disease, *i.e.* causing the clinical symptoms of the disease not to develop in a subject that may be exposed to or predisposed to the disease but does not yet experience or display symptoms of the disease,
(2) inhibiting the disease, *i.e.* arresting or reducing the development of the disease or its clinical symptoms, and
(3) relieving the disease, *i.e.* causing regression of the disease or its clinical symptoms.

The term "effective amount" refers to an amount that may be effective to elicit the desired biological or medical response, including the amount of a compound that, when administered to a subject for treating a disease, is sufficient to effect such treatment for the disease. The effective amount will vary depending on the compound, the disease and its severity and the age, weight, *etc.* of the subject to be treated. The effective amount can include a range of amounts.

### EXAMPLES

The invention will now be illustrated by the following non-limiting examples.

### Example 1 - Emtricitabine/tenofovir alafenamide hemifumarate tablets

The emtricitabine/tenofovir alafenamide hemifumarate formulation was initially developed to a target emtricitabine dose of 200 mg per tablet and target tenofovir alafenamide doses of 25 mg and 40 mg per tablet. Antiviral activity was measured by change in baseline in HIV-1 RNA and DAVG11. Statistically greater reductions in HIV-1 RNA and DAVG11 were observed for the 25 mg tenofovir alafenamide single-agent tablet and the 40 mg tenofovir alafenamide single agent tablet as compared to the tenofovir disoproxil fumarate single-agent tablet, supporting further clinical investigation of 25 mg and 40 mg tenofovir alafenamide.

Emtricitabine/tenofovir alafenamide 200/25 mg and emtricitabine/ tenofovir alafenamide 200/40 mg fixed-dose combination tablet formulations containing 200 mg emtricitabine and 25 mg (tablet A) or 40 mg emtricitabine/tenofovir alafenamide (tablet B) as emtricitabine/tenofovir alafenamide were developed and manufactured for a Phase 1 clinical study. The composition of emtricitabine/tenofovir alafenamide 200/25 mg and 200/40 mg fixed-dose combination tablet formulations evaluated were:

| **Component** | **Tablet A** | **Tablet B** |
|---|---|---|
| | **Emtricitabine/tenofovir alafenamide 200/25 mg (mg/tablet)** | **Emtricitabine/tenofovir alafenamide 200/40 mg (mg/tablet)** |
| **Intragranular** | | |
| Emtricitabine | 200.0 | 200.0 |
| Tenofovir Alafenamide hemifumarate | 28.0 | 44.9 |
| Microcrystalline Cellulose | 179.2 | 162.36 |
| Croscarmellose Sodium | 27.0 | 27.0 |
| Magnesium Stearate | 3.375 | 3.375 |

| **Extragranular** | | |
|---|---|---|
| Croscarmellose Sodium | 9.0 | 9.0 |
| Magnesium Stearate | 3.375 | 3.375 |
| Total Tablet Core Weight | 450 | 450 |

| **Film-Coating** | | |
|---|---|---|
| Opadry II White 85F18422 | 13.5 | 13.5 |

Emtricitabine/tenofovir alafenamide 200/25 mg (tablet A) and 200/40 mg (tablet B) tablets evaluated were manufactured using a dry granulation / tablet compression / film-coating process train. Dry granulation by roller compaction was selected as the means of combining emtricitabine and tenofovir alafenamide in order to minimize exposure of tenofovir alafenamide to moisture during the granulation process. The overall manufacturing process consisted of co-blending and lubricating emtricitabine and tenofovir alafenamide with intragranular excipients, followed by roller compaction and milling. The resulting emtricitabine/tenofovir alafenamide granules were then blended and lubricated with extragranular excipients to produce the emtricitabine/tenofovir alafenamide final powder blend, which was compressed into 450 mg core tablets that were subsequently film-coated with Opadry II White 85F18422.

### Example 2 - stability of emtricitabine/tenofovir alafenamide hemifumarate tablets

The stability of tablets A and B from Example 1 was evaluated at the long-term storage condition of 25 °C/60%RH for 24 months and at the accelerated condition of 40 °C/75%RH for 6 months. Stability results for emtricitabine and tenofovir alafenamide hemifumarate indicated that limited degradation of emtricitabine occurred for either emtricitabine/tenofovir alafenamide hemifumarate tablet strength at any storage condition. After 6 months at 40 °C/75% RH, 4.2% of total tenofovir alafenamide hemifumarate impurities products were observed for tablet A and 3.0% total tenofovir alafenamide hemifumarate impurities products were observed for tablet B.

Dissolution of emtricitabine and tenofovir alafenamide hemifumarate from these tablets did not change. Tablets stored at all conditions exhibited ≥ 98% release of both active agents at all storage times (monitored using USP apparatus II, in 500 ml of 50 mM sodium citrate pH 5.5, at 37 °C and paddle speed of 75 rpm). Moisture contents of these tablets ranged from 1.3 to 2.5% over the course of the stability study. Overall, these stability data demonstrate that tablet A and tablet B packaged in HDPE bottles with 2 g of desiccant remain physically and chemically stable under accelerated conditions (40 °C/75% RH) for 6 months and under long-term storage (25 °C/60% RH) for up to 24 months.

### Example 3 - excipient ranging studies

Formulation development studies were performed by designing, manufacturing, and testing eleven prototype monolayer co-dry granulation emtricitabine/tenofovir alafenamide hemifumarate tablet formulations. These formulations were evaluated for influence of excipient identity and relative composition on tenofovir alafenamide hemifumarate chemical stability. Compositions of the eleven formulations are summarized in the following table:

| **Lot Number** | **A** | **B** | **C** | **D** | **E** | **F** | **G** | **H** | **I** | **J** | **K** |
|---|---|---|---|---|---|---|---|---|---|---|---|
| **Formulation Composition (% w/w)** | | | | | | | | | | | |
| Emtricitabine | 44.44 | 44.44 | 44.44 | 44.44 | 44.44 | 44.44 | 44.44 | 44.44 | 44.44 | 57.14 | 57.14 |
| Tenofovir alafenamide hemifumarate | 2.49 | 2.49 | 2.49 | 2.49 | 2.49 | 2.49 | 2.49 | 6.23 | 6.23 | 3.20 | 8.01 |
| Microcrystalli ne Cellulose | 43.57 | 21.79 | 4.37 | 23.57 | 23.57 | 45.57 | 43.56 | 39.83 | 47.83 | 30.16 | 25.35 |
| Dibasic Calcium Phosphate, Anhydrous | - | 21.79 | 39.20 | - | - | - | - | - | - | - | - |
| Lactose Monohydrate | - | - | - | 20.00 | - | - | - | - | - | - | - |
| Mannitol | - | - | - | - | 20.00 | - | - | - | - | - | - |
| Croscannellos e Sodium | 8.00 | 8.00 | 8.00 | 8.00 | 8.00 | 6.00 | - | 8.00 | - | 8.00 | 8.00 |
| Crospovidone | - | - | - | - | - | - | 8.00 | - | - | - | - |
| Magnesium Stearate | 1.50 | 1.50 | 1.50 | 1.50 | 1.50 | 1.50 | 1.50 | 1.50 | 1.50 | 1.50 | 1.50 |
| **Total Tablet Core Weight (mg)** | **450** | **450** | **450** | **450** | **450** | **450** | **450** | **450** | **450** | **350** | **350** |
| Opadry II Gray 85F97517 | - | - | - | - | - | - | - | 3 | 3 | - | 3 |
| Opadry II Blue 85F105057 | 3 | 3 | 3 | 3 | 3 | 3 | 3 | - | - | 3 | - |

| | | | | | | | | | | | |
|---|---|---|---|---|---|---|---|---|---|---|---|
| 30 tablets were packaged in 60 mL HDPE bottles with 2 g of desiccant and polyester coil. Bottles were induction-sealed with a PP cap. "-" excipient not included in the composition | | | | | | | | | | | |

The following formulation attributes were examined:
- Filler type and excipient matrix composition: microcrystalline cellulose, microcrystalline cellulose and lactose monohydrate, microcrystalline cellulose and mannitol, or microcrystalline cellulose and dibasic calcium phosphate anhydrous.
- Disintegrant type and level: croscarmellose sodium or crospovidone.
- Tenofovir alafenamide hemifumarate drug load: tenofovir alafenamide hemifumarate concentrations of 2.49% and 3.20% w/w in emtricitabine/tenofovir alafenamide hemifumarate 200/10 mg tablets and tenofovir alafenamide hemifumarate concentrations of 6.23% and 8.01% w/w in emtricitabine/tenofovir alafenamide 200/25 mg tablets.

All film-coated tablets were packaged as a 30 count configuration in 60 mL HDPE bottle with 2 grams of silica gel desiccant and a polyester coil. HDPE bottles were induction-sealed using a polypropylene (PP) cap with an aluminum-faced liner. Chemical stability was monitored over 3 months at 40 °C/75% RH. For the 5 formulations tested (batches A-E), the total tenofovir alafenamide hemifumarate degradation products (compared to initial) increased by 0.7 to 1.7% after 1 month and by 2.3 to 2.7% after 3 months. Overall, the filler system did not significantly influence tenofovir alafenamide hemifumarate degradation after 3 months at the accelerated conditions.

### Example 4 - effect of tenofovir alafenamide hemifumarate loading on stability in emtricitabine/tenofovir alafenamide hemifumarate tablets

The impact of tenofovir alafenamide hemifumarate drug load on tenofovir alafenamide hemifumarate stability in emtricitabine/tenofovir alafenamide 200/10 mg and 200/25 mg tablets was evaluated using a range of tenofovir alafenamide hemifumarate drug loads from 2.49% to 8.01% with concomitant adjustment in microcrystalline cellulose content. Emtricitabine/tenofovir alafenamide 200/10 mg tablet formulations contained 2.49% w/w tenofovir alafenamide hemifumarate or 3.20% w/w tenofovir alafenamide hemifumarate, while emtricitabine/tenofovir alafenamide 200/25 mg tablet formulations contained 6.23% w/w tenofovir alafenamide hemifumarate or 8.01% w/w tenofovir alafenamide hemifumarate. Higher drug loads were achieved by reducing the total tablet weight from 450 mg to 350 mg.

Tenofovir alafenamide hemifumarate chemical stability as a function of drug load is summarized in the table below:

| **Tenofovir alafenamide hemifumarate concentration (% w/w)** | **Tenofovir alafenamide hemifumarate 2.49%** | | | **Tenofovir alafenamide hemifumarate 3.20%** | | | | **Tenofovir alafenamide hemifumarate 6.23%** | | | **Tenofovir alafenamide hemifumarate 8.01%** | | | |
|---|---|---|---|---|---|---|---|---|---|---|---|---|---|---|
| **Lot Number** | **A** | | | **J** | | | | **H** | | | **K** | | | |
| **Condition: 40°C/75% RH** | **Time Point (months)** | | | **Time Point (months)** | | | | **Time Point (months)** | | | **Time Point (months)** | | | |
| | **0** | **1** | **3** | **0** | **1** | **3** | **6** | **0** | **1** | **3** | **0** | **1** | **3** | **6** |
| Tenofovir alafenamide hemifumarate Degradation Products (%) | | | | | | | | | | | | | | |
| PMPA | 0.22 | 0.60 | 1.32 | 0.26 | 0.49 | 0.82 | 2.04 | 0.23 | 0.49 | 1.00 | 0.21 | 0.48 | 0.77 | 1.33 |
| PMPA Anhydride | 0.29 | 0.43 | 1.21 | 0.29 | 0.37 | 0.76 | 1.97 | 0.28 | 0.37 | 0.85 | 0.27 | 0.37 | 0.67 | 1.24 |
| Monophenyl PMPA | nd | 0.06 | 0.07 | 0.05 | 0.05 | 0.04 | 0.13 | 0.06 | 0.05 | 0.07 | nd | 0.06 | 0.07 | 0.10 |
| PMPA Monoamidate | 0.08 | 0.11 | 0.14 | 0.10 | 0.11 | 0.13 | 0.18 | 0.06 | 0.08 | 0.11 | 0.07 | 0.14 | 0.16 | 0.09 |
| Phenol | nd | nd | 0.07 | nd | nd | nd | 0.08 | nd | tr | 0.07 | 0.09 | tr | 0.05 | 0.07 |
| Unspecified^{b} | nd | 0.11 | 0.16 | nd | nd | 0.12 | 0.22 | nd | 0.05 | 0.07 | nd | 0.06 | nd | 0.11 |
| Total tenofovir alafenamide hemifumarate Deg. (%) | 0.6 | 1.3 | 3.0 | 0.7 | 1.0 | 1.8 | 4.6 | 0.6 | 1.0 | 2.2 | 0.6 | 1.1 | 1.7 | 2.9 |

| | | | | | | | | | | | | | | |
|---|---|---|---|---|---|---|---|---|---|---|---|---|---|---|
| nd: not detected (< 0.025%) tr: trace (0.025% < impurity < 0.05%) ^{a} 30 tablets were packaged in 60 mL HDPE bottles with 2 g of desiccant and polyester coil. Bottles were induction-sealed with a PP cap. ^{b} represents sum of all unspecified degradation products/impurities | | | | | | | | | | | | | | |

Emtricitabine/tenofovir alafenamide 200/10 mg tablets containing 2.49% w/w tenofovir alafenamide hemifumarate exhibited increases in total tenofovir alafenamide hemifumarate degradation products of 0.7% and 2.4% after 1 and 3 months, respectively. Emtricitabine/tenofovir alafenamide 200/10 mg tablets containing 3.20% w/w tenofovir alafenamide hemifumarate exhibited increases in total tenofovir alafenamide hemifumarate degradation products of 0.3% and 1.1% after 1 and 3 months, respectively. Increasing the tenofovir alafenamide hemifumarate drug load from 2.49% w/w to 3.20% w/w tenofovir alafenamide hemifumarate resulted in a 50% reduction in total tenofovir alafenamide hemifumarate degradation products after 3 months under accelerated conditions.

Emtricitabine/tenofovir alafenamide 200/25 mg tablets containing 8.01% w/w tenofovir alafenamide hemifumarate demonstrated better tenofovir alafenamide hemifumarate chemical stability than tablets containing 6.23% w/w tenofovir alafenamide hemifumarate. After 3 months, total tenofovir alafenamide hemifumarate degradation products in emtricitabine/tenofovir alafenamide 200/25 mg tablets increased by 1.5% for the 6.23% w/w tenofovir alafenamide hemifumarate formulation and 1.1% for the 8.01% w/w tenofovir alafenamide hemifumarate formulation. Based on the results of the tenofovir alafenamide hemifumarate drug load study, tenofovir alafenamide hemifumarate contents of 3.20% w/w and 8.01% w/w were selected for the emtricitabine/tenofovir alafenamide 200/10 mg and 200/25 mg fixed dose combination tablets, respectively.

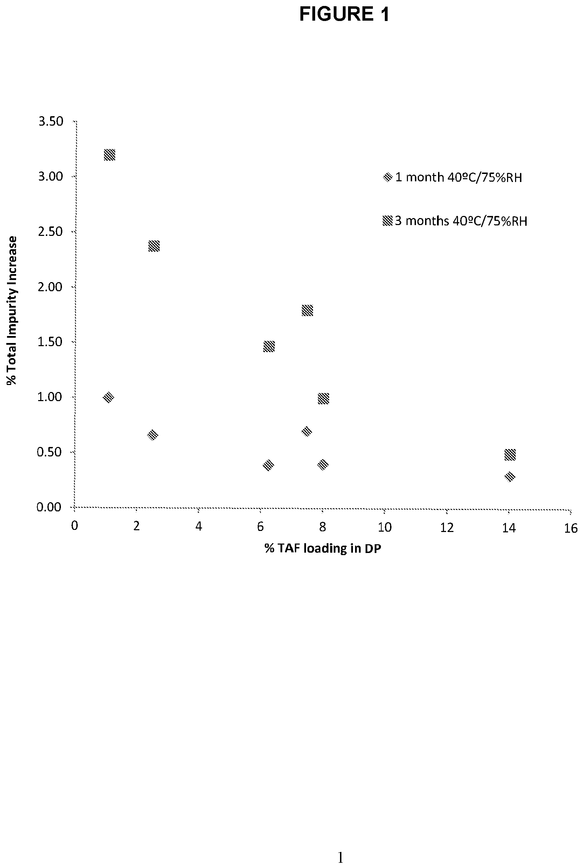

Figure 1 shows a plot of the increase in tenofovir alafenamide hemifumarate-related degradation products as a function of tenofovir alafenamide hemifumarate loading at 1 month and 3 months (at 40 °C/75% RH).

### Example 5

As a result of the excipient and drug load evaluations, two formulations (emtricitabine/tenofovir alafenamide 200/10 mg, tablet C; and emtricitabine/tenofovir alafenamide 200/25 mg, tablet D) were developed for use in further studies. The compositions of these formulations shown in the following table:

| **Component** | **Tablet C** | **Tablet D** |
|---|---|---|
| | **Emtricitabine/tenofovir alafenamide 200/10 mg (mg/tablet)** | **Emtricitabine/tenofovir alafenamide 200/25 mg (mg/tablet)** |
| **Intragranular** | | |
| Emtricitabine | 200.00 | 200.00 |
| Tenofovir Alafenamide Hemifumarate | 11.20 | 28.00 |
| Microcrystalline Cellulose | 105.56 | 88.70 |
| Croscarmellose Sodium | 28.00 | 28.00 |
| Magnesium Stearate | 2.625 | 2.625 |

| **Extragranular** | | |
|---|---|---|
| Magnesium Stearate | 2.625 | 2.625 |
| **Total Tablet Core Weight** | **350** | **350** |

| **Film-Coating** | | |
|---|---|---|
| Opadry II Gray 85F97517 | 10.5 | -- |
| Opadry II Blue 85F105057 | -- | 10.5 |

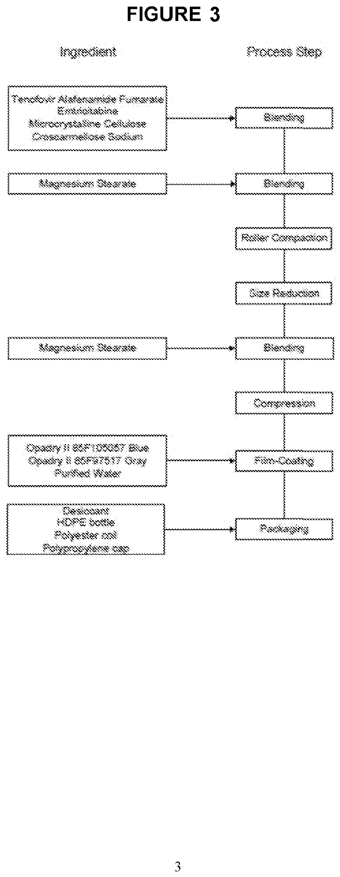

Emtricitabine and tenofovir alafenamide hemifumarate were co-blended with microcrystalline cellulose and croscarmellose sodium, followed by lubrication with magnesium stearate. The roller compaction preblend was then roller compacted and milled using an oscillating mill. The resultant granules were lubricated with magnesium stearate and compressed into 350 mg tablet cores which that were subsequently film coated. A flow diagram illustrating the process by which tablets C and D were prepared is shown in Figure 3.

### Example 6

Tenofovir alafenamide hemifumarate undergoes solid-state hydrolysis and therefore the inclusion of desiccant in the primary package is included to control the level of moisture in emtricitabine/tenofovir alafenamide hemifumarate tablets. Packaging development was performed on tablets C and D to evaluate the impact of desiccant amount on the chemical stability of tenofovir alafenamide hemifumarate in emtricitabine/tenofovir alafenamide hemifumarate tablets during storage.

Tablets C and D were packaged at 30 count in 60 mL HDPE bottles with either 2 or 3 grams of desiccant and a polyester coil, and sealed with an induction seal. Chemical stability was monitored for up to 6 months at 40 °C/75% RH.

Tenofovir alafenamide hemifumarate-related total degradation products in tablet C after 6 months under accelerated conditions were 3.9% and 3.3% for bottles packaged with 2 g and 3 g of desiccant, respectively. In comparison, Tenofovir alafenamide hemifumarate-related total degradation products in tablet D were 2.3% and 2.4% for bottles containing 2 g and 3 g desiccant, respectively.

### Example 7 - Emtricitabine/tenofovir alafenamide hemifumarate bioequivalence studies

Randomized, open-label, single-dose, 2-way, crossover studies were performed to determine:
- *Study 1:* the bioequivalence of emtricitabine and TAF, administered as F/TAF fixed-dose combination tablet (tablet C) simultaneously with elvitegravir and cobicistat or as E/C/F/TAF fixed-dose combination tablet.
- *Study 2:* the bioequivalence of emtricitabine and TAF, administered as F/TAF fixed-dose combination tablet (tablet D) or as E/C/F/TAF fixed-dose combination tablet.
- *Study 3:* the bioequivalence of emtricitabine and TAF, administered as F/TAF fixed-dose combination tablet (tablet D) and emtricitabine and TAF from co-administration of Emtriva^{®} (emtricitabine) capsules and TAF single-agent tablets.

### Duration of treatment

Subjects were randomized to one of two treatment sequences (AB or BA) and received a single dose of one of the following treatments (A or B) on days 1 and 7 (studies 1 and 2) or days 1 and 15 (study 3):

### Study 1

**Treatment A:** Single dose of F/TAF (200/10 mg) fixed-dose combination tablet (tablet C) administered simultaneously with EVG 150 mg and COBI 150 mg tablets orally under fed conditions.

**Treatment B:** Single dose of E/C/F/TAF (150/150/200/10 mg) fixed-dose combination tablet administered orally under fed conditions.

### Study 2

**Treatment A:** Single dose of F/TAF (200/25 mg) fixed-dose combination tablet (tablet D) administered orally under fed conditions.

**Treatment B:** Single dose of E/C/F/TAF (150/150/200/10 mg) fixed-dose combination tablet administered orally under fed conditions.

### Study 3

**Treatment A:** Single dose of F/TAF (200/25 mg) fixed-dose combination tablet (tablet D) administered orally under fed conditions.

**Treatment B:** Single dose of Emtriva^{®} 200 mg capsule plus a single TAF 25 mg tablet administered orally under fed conditions.

*Studies 1 and 2:* In each study, the total duration of the study was 22 days. There was a 6 day washout period between dosing on days 1 and 7, and a 14 day follow-up period.

*Study 3:* The total duration of the study was 29 days. Doses were administered on days 1 and 15 and there was a 14 day follow-up period.

### Criteria for Evaluation

The following plasma pharmacokinetic parameters were calculated: Cₘₐₓ, Tₘₐₓ, Cₗₐₛₜ, t_{1/2}, AUCₗₐₛₜ, AUC_{inf}, %AUCₑₓₚ, V_{z}/F, CL/F.

### Statistical Methods

### Studies 1 and 2

Pharmacokinetics: Plasma concentrations and PK parameters were listed and summarized by treatment group using descriptive statistics. In addition, a parametric analysis of variance using a mixed-effects model appropriate for a crossover design was fitted to the natural logarithmic transformation of the PK parameters (AUC_{inf}, AUCₗₐₛₜ, and Cₘₐₓ). Two-sided 90% confidence intervals (CIs) were constructed for the ratio of geometric least-squares means (GLSMs) of each PK parameter for emtricitabine and tenofovir alafenamide hemifumarate. Bioequivalence of emtricitabine and tenofovir alafenamide hemifumarate in the F/TAF fixed-dose combination (tablet C/tablet D) to the emtricitabine and tenofovir alafenamide hemifumarate components in E/C/F/TAF fixed-dose combination was concluded if the 90% CI of the GLSM (geometric least-squares mean) ratio of the pharmacokinetic parameters for each analyte between two formulations fell within the prespecified bioequivalence boundary of 80% to 125%.

### Study 3

Pharmacokinetics: Plasma concentrations and PK parameters were listed and summarized by treatment group using descriptive statistics. In addition, the geometric mean, 95% confidence interval (CI) and the mean and standard deviation (SD) of the natural-log transformed values were presented for individual subject PK parameter data.

For evaluation of bioequivalence of F/TAF and FTC + TAF, the PK parameters (natural-log transformed AUC_{inf}, AUCₗₐₛₜ, and Cₘₐₓ) were compared. A parametric analysis of variance using a mixed-effects model was filled to the natural log-transformed values of PK parameters. The 90% CIs were provided for the geometric mean ratios (GMRs) of PK parameters between pairs of interest. It was concluded that the formulations were bioequivalent if the 90% CIs of the GMRs for selected PK parameters (i.e., AUC_{inf}, AUCₗₐₛₜ, and Cₘₐₓ) fell within the prespecified bioequivalence boundary of 80% to 125%.

### Results

### Study 1

A total of 100 patients were randomized and received at least one dose of study drug. 98 patients completed the study.

Statistical comparisons of emtricitabine and TAF pharmacokinetic parameters between F/TAF 200/10 mg (tablet C) co-administered with EVG 150 mg and COBI 150 mg and E/C/F/TAF are shown in the table below:

| **TAF PK Parameter** | **N** | **Test Mean (CV%)** | **N** | **Reference Mean (CV%)** | **GLSM Ratio (Test/Reference) (%)** | **90% CI (%)** |
|---|---|---|---|---|---|---|
| **F/TAF (200/10 mg) + E + C (Test) vs E/C/F/TAF (150/150/200/10 mg) (Reference)** | | | | | | |
| AUCₗₐₛₜ (h•ng/mL) | 97 | 336.6 (33.9) | 99 | 340.2 (33.8) | 97.96 | 94.69, 101.34 |
| AUC_{inf} (h•ng/mL) | 97 | 351.8 (31.0) | 99 | 354.1 (32.9) | 98.34 | 94.81, 101.99 |
| Cₘₐₓ (ng/mL) | 97 | 301.6 (48.8) | 99 | 310.3 (48.7) | 96.86 | 89.36, 104.99 |

| **Emtricitabine PK Parameter** | **N** | **Test Mean (CV%)** | **N** | **Reference Mean (CV%)** | **GLSM Ratio (Test/Reference) (%)** | **90% CI (%)** |
|---|---|---|---|---|---|---|
| **F/TAF (200/10 mg) + E + C (Test) vs E/C/F/TAF (150/150/200/10 mg) (Reference)** | | | | | | |
| AUCₗₐₛₜ (h•ng/mL) | 97 | 10159.2 (17.2) | 99 | 10086.8 (15.9) | 99.84 | 98.41, 101.29 |
| AUC_{inf} (h•ng/mL) | 97 | 10535.1 (27.0) | 99 | 10294.4 (15.8) | 100.67 | 98.24, 103.16 |
| Cₘₐₓ (ng/mL) | 97 | 1660.8 (20.6) | 99 | 1662.6 (19.1) | 99.57 | 96.78, 102.44 |

The GLSM ratios and corresponding 90% CIs of AUCₗₐₛₜ, AUC_{inf}, and Cₘₐₓ for emtricitabine and tenofovir alafenamide were contained within the 80% to 125% boundary criteria specified for bioequivalence.

### Study 2

A total of 56 patients were randomized and received at least one dose of study drug. 54 patients completed the study.

Statistical comparisons of emtricitabine and TAF pharmacokinetic parameters between F/TAF 200/25 mg (tablet D) and E/C/F/TAF are shown in the table below:

| **TAF PK Parameter** | **N** | **Test Mean (CV%)** | **N** | **Reference Mean (CV%)** | **GLSM Ratio (Test/Reference) (%)** | **90% CI (%)** |
|---|---|---|---|---|---|---|
| **F/TAF (200/25 mg) vs E/C/F/TAF (150/150/200/10 mg) (Reference)** | | | | | | |
| AUCₗₐₛₜ (h•ng/mL) | 116 | 374.0 (43.4) | 116 | 369.3 (40.6) | 100.32 | 96.48, 104.31 |
| AUC_{inf} (h•ng/mL) | 95 | 396.4 (42.6) | 97 | 389.5 (39.3) | 98.54 | 94.61, 102.62 |
| Cₘₐₓ (ng/mL) | 116 | 280.5 (62.9) | 116 | 267.8 (59.8) | 103.63 | 95.46, 112.49 |

| **Emtricitabine PK Parameter** | **N** | **Test Mean (CV%)** | **N** | **Reference Mean (CV%)** | **GLSM Ratio (Test/Reference) (%)** | **90% CI (%)** |
|---|---|---|---|---|---|---|
| **F/TAF (200/25 mg) vs E/C/F/TAF (150/150/200/10 mg) (Reference)** | | | | | | |
| AUCₗₐₛₜ (h•ng/mL) | 116 | 9423.9 (19.3) | 116 | 10475.3 (19.7) | 90.01 | 88.88, 91.16 |
| AUC_{inf} (h•ng/mL) | 116 | 9654.6 (19.3) | 116 | 10706.6 (19.6) | 90.20 | 89.06, 91.35 |
| Cₘₐₓ (ng/mL) | 116 | 1577.4 (26.8) | 116 | 1601.7 (19.6) | 97.26 | 94.57, 100.03 |

The GLSM ratios and corresponding 90% CIs of AUCₗₐₛₜ, AUC_{inf}, and Cₘₐₓ for emtricitabine and tenofovir alafenamide were contained within the 80% to 125% boundary criteria specified for bioequivalence.

### Study 3

A total of 116 patients were randomized, received at least one dose of study drug and completed the study.

Statistical comparisons of emtricitabine and TAF pharmacokinetic parameters between F/TAF 200/25 mg (tablet D) and Emtriva^{®} 200 mg capsules co-administered with TAF 25 mg tablets are shown in the table below:

| | **GLSMs by treatment** | | **GLSM Ratio (Test/Reference) (%)** | **90% CI (%)** |
|---|---|---|---|---|
| | **Test (F/TAF) Mean (N=55)** | **Reference (FTC + TAF) Mean (N=55)** | | |
| **TAF PK Parameter** | | | | |
| AUCₗₐₛₜ (h•ng/mL) | 245.91 | 239.48 | 102.68 | 95.78, 110.09 |
| AUC_{inf} (h•ng/mL) | 254.18^{a} | 240.33^{b} | 105.77 | 97.26, 115.01 |
| Cₘₐₓ (ng/mL) | 209.36 | 226.11 | 92.59 | 82.31, 104.16 |

| | **Test (F/TAF) Mean (CV%)** | **Reference (FTC + TAF) Mean (CV%)** | **GLSM Ratio (Test/Reference) (%)** | **90% CI (%)** |
|---|---|---|---|---|
| **Emtricitabine PK Parameter** | | | | |
| AUCₗₐₛₜ (h•ng/mL) | 9049.70 | 9410.78 | 96.16 | 94.29, 98.08 |
| AUC_{inf} (h•ng/mL) | 9259.49 | 9636.68 | 96.09 | 94.24, 97.96 |
| Cₘₐₓ (ng/mL) | 1813.87 | 1727.84 | 104.98 | 100.75, 109.39 |
| a N=43 | | | | |
| b N=48 | | | | |

The GLSM ratios and corresponding 90% CIs of AUCₗₐₛₜ, AUC_{inf}, and Cₘₐₓ for emtricitabine and tenofovir alafenamide were contained within the 80% to 125% boundary criteria specified for bioequivalence.

### Conclusions

These studies demonstrate that:
- Emtricitabine and TAF from F/TAF 200/10 mg (tablet C) co-administered with EVG and COBI single-agent tablets were bioequivalent to the exposures of emtricitabine and TAF from the reference treatment of E/C/F/TAF.
- Emtricitabine and TAF from F/TAF 200/25 mg (tablet D) were bioequivalent to the exposures of emtricitabine and TAF from the reference treatment of E/C/F/TAF.
- Emtricitabine and TAF from F/TAF 200/25 mg (tablet D) were bioequivalent to the exposures of emtricitabine and TAF from the reference treatment of the co-administration of an Emtriva^{®} capsule 200mg, co-administered with a TAF single-agent 25 mg tablet.

### Example 8 - Tenofovir alafenamide hemifumarate stability studies

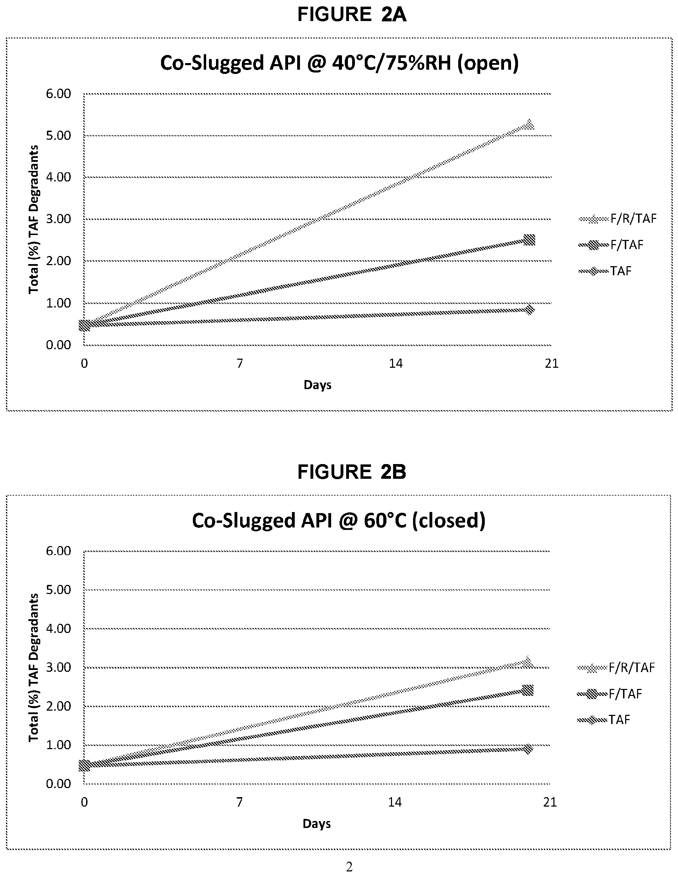

Studies were conducted to assess the stability of tenofovir alafenamide hemifumarate in the presence of (a) emtricitabine, and (b) emtricitabine and rilpivirine HCl. These data are presented in Figures 2A and B. Figure 2A shows the total degradation of tenofovir alafenamide hemifumarate at 40°C/75% RH in open conditions (i.e., unsealed containers with no desiccant present). Figure 2B shows the total degradation of tenofovir alafenamide hemifumarate at 60°C in closed conditions. These data show that the rate of degradation of tenofovir alafenamide hemifumarate is increased in the presence of emtricitabine, and is further increased in the presence of both emtricitabine and rilpivirine HCl.

### Example 9 - Long Term Stability Studies

The long term stability of Tablets C and D was measured over the course of 12 months at 30°C/75% relative humidity (RH). The results of those studies are provided in the following tables:

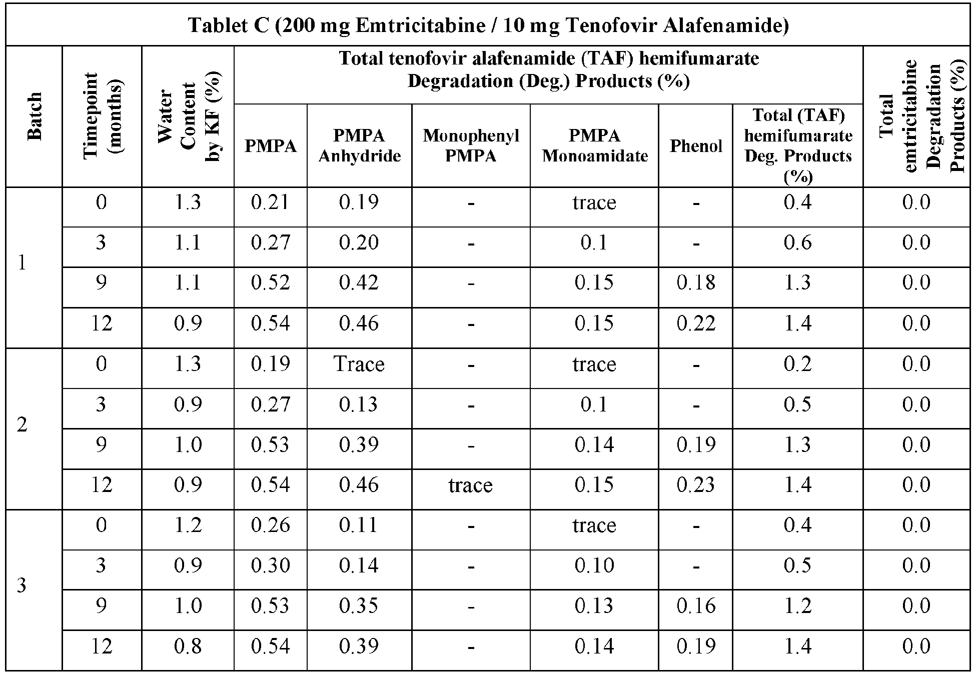

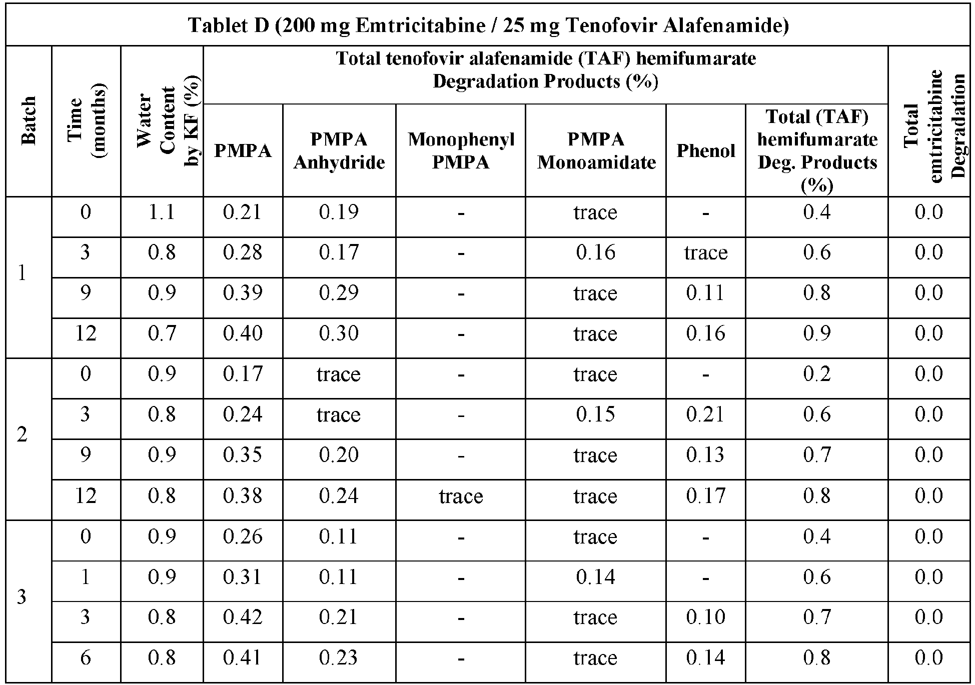

These results demonstrate that TAF in the tenofovir alafenamide hemifumarate tablets (packaged in induction sealed, 60 mL HDPE bottles (30 tablets/bottle) with 3 g of desiccant) is stable under long term storage conditions (30°C/75% RH).

The invention has been described with reference to various specific and preferred embodiments and techniques. However, it should be understood that many variations and modifications may be made while remaining within the scope of the appended claims.

## Claims

1. A tablet comprising, as the only active ingredients, an amount of emtricitabine or a pharmaceutically acceptable salt thereof which provides the same amount of emtricitabine as 200 mg of emtricitabine free base, and an amount of tenofovir alafenamide or a pharmaceutically acceptable salt thereof which provides the same amount of tenofovir alafenamide as 25 mg of tenofovir alafenamide free base, wherein the proportion of tenofovir alafenamide in the tablet is from 4% to 9% by weight.

2. The tablet of claim 1, wherein the tablet comprises 7-9% by weight tenofovir alafenamide, preferably 8% by weight tenofovir alafenamide.

3. The tablet of claim 1 or 2, wherein the tenofovir alafenamide is associated with fumarate, such as monofumarate or hemifumarate.

4. The tablet of any of claims 1-3, wherein the tenofovir alafenamide is in the form of tenofovir alafenamide hemifumarate.

5. The tablet of any of claims 1-4, wherein the tablet comprises less than 300 mg of excipients.

6. The tablet of any of claims 1-5, wherein the tablet includes lactose and/or microcrystalline cellulose.

7. The tablet of claim 6, wherein the tablet includes less than 250 mg lactose.

8. The tablet of any of claims 1-5, wherein the tablet comprises microcrystalline cellulose and croscarmellose sodium.

9. The tablet of claim 8, wherein the tablet comprises microcrystalline cellulose, croscarmellose sodium and magnesium stearate.

10. The tablet of any of claims 1-9, wherein the tablet is a monolayer tablet.

11. The tablet of any of claims 1-10, wherein the tablet is coated with a film coating, wherein the amount of the coating is 2-4% by weight of the core of the tablet.

12. A kit comprising a) the tablet according to any one of claims 1-11 and b) a desiccant, preferably wherein said desiccant is silica gel.

13. The tablet of any one of claims 1-11 for use in a method of preventing or treating an HIV infection.

14. The tablet for use according to claim 13, wherein the tablet is administered once daily.

15. The tablet for use according to claim 14, wherein the tablet is administered prior to and following an event that would increase the individual's risk of acquiring HIV.

## Patentansprüche

1. Tablette, umfassend als einzige Wirkstoffe eine Menge von Emtricitabin oder einem pharmazeutisch unbedenklichen Salz davon, die die gleiche Menge von Emtricitabin wie 200 mg Emtricitabin freie Base bereitstellt, und eine Menge von Tenofoviralafenamid oder einem pharmazeutisch unbedenklichen Salz davon, die die gleiche Menge Tenofoviralafenamid wie 25 mg Tenofoviralafenamid freie Base bereitstellt, wobei der Anteil von Tenofoviralafenamid in der Tablette 4 Gew.-% bis 9 Gew.-% beträgt.

2. Tablette nach Anspruch 1, wobei die Tablette 7-9 Gew.-% Tenofoviralafenamid, vorzugsweise 8 Gew.-% Tenofoviralafenamid umfasst.

3. Tablette nach Anspruch 1 oder 2, wobei das Tenofoviralafenamid mit Fumarat, wie Monofumarat oder Hemifumarat, assoziiert ist.

4. Tablette nach einem der Ansprüche 1-3, wobei das Tenofoviralafenamid in Form von Tenofoviralafenamid-Hemifumarat vorliegt.

5. Tablette nach einem der Ansprüche 1-4, wobei die Tablette weniger als 300 mg an Exzipienten umfasst.

6. Tablette nach einem der Ansprüche 1-5, wobei die Tablette Lactose und/oder mikrokristalline Cellulose enthält.

7. Tablette nach Anspruch 6, wobei die Tablette weniger als 250 mg Lactose enthält.

8. Tablette nach einem der Ansprüche 1-5, wobei die Tablette mikrokristalline Cellulose und Croscarmellose-Natrium umfasst.

9. Tablette nach Anspruch 8, wobei die Tablette mikrokristalline Cellulose, Croscarmellose-Natrium und Magnesiumstearat umfasst.

10. Tablette nach einem der Ansprüche 1-9, wobei es sich bei der Tablette um eine einschichtige Tablette handelt.

11. Tablette nach einem der Ansprüche 1-10, wobei die Tablette mit einer Filmbeschichtung beschichtet ist, wobei die Menge der Beschichtung 2 bis 4 Gew.-% bezogen auf den Kern der Tablette beträgt.

12. Kit, umfassend a) die Tablette nach einem der Ansprüche 1-11 und b) ein Trockenmittel, wobei das Trockenmittel vorzugsweise Kieselgel ist.

13. Tablette nach einem der Ansprüche 1-11 zur Verwendung bei einem Verfahren zur Prävention einer HIV-Infektion.

14. Tablette zur Verwendung nach Anspruch 13, wobei die Tablette einmal täglich verabreicht wird.

15. Tablette zur Verwendung nach Anspruch 14, wobei die Tablette vor und nach einem Ereignis, das das Risiko des Individuums, HIV zu erwerben, erhöht, verabreicht wird.

## Revendications

1. Comprimé comprenant, comme seuls principes actifs, une quantité d'emtricitabine ou d'un sel pharmaceutiquement acceptable de celle-ci qui fournit la même quantité d'emtricitabine que 200 mg d'emtricitabine base libre et une quantité de ténofovir alafénamide ou d'un sel pharmaceutiquement acceptable de celui-ci qui fournit la même quantité de ténofovir alafénamide que 25 mg de ténofovir alafénamide base libre, dans lequel la proportion de ténofovir alafénamide dans le comprimé va de 4 % à 9 % en poids.

2. Comprimé selon la revendication 1, dans lequel le comprimé comprend 7 à 9 % en poids de ténofovir alafénamide, de préférence 8 % en poids de ténofovir alafénamide.

3. Comprimé selon la revendication 1 ou 2, dans lequel le ténofovir alafénamide est associé au fumarate, tel que le monofumarate ou l'hémifumarate.

4. Comprimé selon l'une quelconque des revendications 1 à 3, dans lequel le ténofovir alafénamide est sous la forme d'hémifumarate de ténofovir alafénamide.

5. Comprimé selon l'une quelconque des revendications 1 à 4, dans lequel le comprimé comprend moins de 300 mg d'excipients.

6. Comprimé selon l'une quelconque des revendications 1 à 5, dans lequel le comprimé renferme du lactose et/ou de la cellulose microcristalline.

7. Comprimé selon la revendication 6, dans lequel le comprimé renferme moins de 250 mg de lactose.

8. Comprimé selon l'une quelconque des revendications 1 à 5, dans lequel le comprimé comprend de la cellulose microcristalline et de la croscarmellose sodique.

9. Comprimé selon la revendication 8, dans lequel le comprimé comprend de la cellulose microcristalline, de la croscarmellose sodique et du stéarate de magnésium.

10. Comprimé selon l'une quelconque des revendications 1 à 9, dans lequel le comprimé est un comprimé monocouche.

11. Comprimé selon l'une quelconque des revendications 1 à 10, dans lequel le comprimé est enrobé d'un enrobage pelliculaire, dans lequel la quantité de l'enrobage est de 2 à 4 % en poids du noyau du comprimé.

12. Kit comprenant a) le comprimé selon l'une quelconque des revendications 1 à 11 et b) un déshydratant, de préférence dans lequel ledit déshydratant est du gel de silice.

13. Comprimé selon l'une quelconque des revendications 1 à 11 destiné à être utilisé dans un procédé de prévention ou de traitement d'une infection par le VIH.

14. Comprimé destiné à être utilisé selon la revendication 13, dans lequel le comprimé est administré une fois par jour.

15. Comprimé destiné à être utilisé selon la revendication 14, dans lequel le comprimé est administré avant et après un événement qui augmenterait le risque pour l'individu de contracter le VIH.
